# EUROPEAN PATENT APPLICATION

(11) **EP 4 785 955 A1**
(43) Date of publication of application: **05.08.2026**
(21) Application number: 24873069.9
(22) Date of filing: 27.09.2024
(51) Int. Cl.: A61K 39/395, A61K 9/08, A61K 47/22, A61K 47/02, A61K 47/12, A61K 47/26, A61K 47/18, A61K 9/00, A61P 17/06, A61P 19/02

(54) **STABLE LIQUID FORMULATION OF ANTI-IL-23 ANTIBODY**

(30) Priority: 27.09.2023 KR 20230130943
(71) Applicant: Samsung Bioepis Co., Ltd., Yeonsu-gu Incheon 21987 (KR)
(72) Inventor: OH, In Young, Incheon 21971 (KR); JUNG, So Yun, Incheon 21986 (KR); JEONG, Woo Jin, Seoul 07531 (KR); PARK, Ji Eun, Incheon 21982 (KR); HWANG, Ji Young, Incheon 21998 (KR); KIM, In Ae, Seoul 05090 (KR); LEE, Hun Joo, Incheon 21996 (KR); CHANG, Sung Keun, Incheon 22003 (KR)
(74) Representative: Boult Wade Tennant LLP
(86) International application number: PCT/KR2024/014793
(87) International publication number: WO 2025/071362

(57) **Abstract**

The present disclosure relates to: a liquid formulation comprising an anti-IL-23 antibody, a buffer, and a stabilizer, having a pH of about 4.0 to about 8.5; a device comprising the same; and use thereof for treating an IL-23-related condition. The liquid formulation exhibits excellent stability, and thus may be advantageously used as a pharmaceutical product for treating IL-23-related conditions.

## Description

### Technical Field

The present disclosure relates to a stable liquid formulation of an anti-IL-23 antibody, a device comprising the same, and a use thereof for treating an IL-23-related condition.

### Background Art

Antibody pharmaceuticals have a higher molecular weight than general protein pharmaceuticals and possess complex secondary and higher-order structures, which may lead to problems of physicochemical instability. For this reason, antibody pharmaceuticals require the development of optimal formulations that ensure quality and stability throughout the entire process of manufacturing, storage, and administration to patients. Protein instability may originate from various external factors such as temperature, light, and chemical factors, which may lead to a decrease in activity and efficacy, or cause immunogenicity when administered to the human body. Therefore, it is important to improve such instability of antibody pharmaceuticals so as to maintain optimal quality until administration to patients. To achieve optimal quality, methods such as changing the buffer solution, evaluating the optimal pH, and adding stabilizers are employed. Since each antibody protein has different substance characteristics and the optimal combination of buffer solution, pH, and stabilizer may vary, there is a need to derive a formulation suitable for a target substance to solve these issues.

For example, guselkumab, an IL-23 inhibitor, is composed of a liquid formulation containing 100 mg/mL anti-IL-23 IgG, 11 mM histidine, 7.9% (w/v) sucrose, and 0.05% (w/v) polysorbate 80, which is manufactured and sold under the trade name Tremfya^{®}. To stabilize liquid formulations containing various antibody proteins such as guselkumab, various conditions-including the types and concentrations of components such as buffers, stabilizers, and surfactants, as well as the application of specific pH levels-are being reviewed and implemented, as seen in the commercial composition of Tremfya^{®}.

However, in order to confirm the stability of a protein until it is administered to a patient, it is necessary to evaluate major quality attributes under storage temperature conditions throughout the expiration period, requiring a long period of time to verify the developed formulation. To effectively evaluate the maintenance of protein stability and the optimal formulation in a short period, an optimal formulation combination may be selected by analyzing new quality indicators after exposure to factors that cause structural and physicochemical denaturation of proteins, such as heat (temperature) and chemical substances.

### Disclosure

### Technical Problem

Accordingly, the present disclosure relates to a stable liquid formulation of an anti-IL-23 antibody such as guselkumab. Specifically, by applying a formulation method that minimizes physicochemical changes of an anti-IL-23 antibody such as guselkumab, the present disclosure provides a formulation having increased stability compared to a commercially available formulation of an anti-IL-23 antibody such as guselkumab (e.g., Tremfya^{®}).

An aspect is to provide a liquid formulation comprising: an anti-IL-23 antibody; a buffer; and a stabilizer comprising at least one selected from sugars, sugar alcohols, amino acids, metal salts, salts thereof and hydrates thereof; wherein the pH is about 4.0 to about 8.5.

Another aspect is to provide a device comprising the liquid formulation.

Another aspect is to provide a method of treating an IL-23 related condition, comprising administering the liquid formulation to a subject in need thereof.

Another aspect is to provide a use of the liquid formulation in the manufacture of a medicament for treating an IL-23 related condition.

### Technical Solution

All technical terms used herein, unless otherwise defined, have the same meaning as commonly understood by those skilled in the art to which the present disclosure belongs. Additionally, although preferred methods and samples are described herein, similar or equivalent methods and samples are also included in the scope of the present disclosure. Additionally, numerical values described herein are considered to include the meaning of "about" even if not explicitly stated. The contents of all publications cited as references herein are incorporated herein by reference in their entirety.

The terms "about" or "approximately" as used herein may generally be interpreted to include values or ranges within 10%, within 5%, within 4%, within 3%, within 2%, or within 1% above or below a given value or range.

An aspect provides a stable liquid formulation of anti-IL-23 antibodies, specifically, provides a liquid formulation comprising:
an anti-IL-23 antibody;
a buffer; and
a stabilizer comprising at least one selected from sugars, sugar alcohols, amino acids, metal salts, salts thereof, and hydrates thereof;
wherein the pH is about 4.0 to about 8.5.

### (1) Antibody

As used herein, the term "antibody" may be interpreted to comprise a full-length antibody or an antigen-binding fragment thereof. The antibody comprises monoclonal antibodies, polyclonal antibodies, humanized antibodies, human antibodies and chimeric antibodies.

The term "antigen-binding fragment" refers to a fragment including an antigen-binding site of an antibody. For example, the antigen-binding fragment comprises, but is not limited to, a Fab fragment, an F(ab')₂ fragment, an Fc fragment, or an scFv fragment.

As used herein, the antibody may be an anti-IL-23 antibody. The anti-IL-23 antibody may refer to any antibody that specifically binds to interleukin-23 (IL-23). IL-23 has a heterodimer structure in which an IL-23p19 subunit and an IL-12p40 subunit are linked by a SS-bond. The IL-23 heterodimers bind to a receptor complex, specifically, the p19 subunit binds to IL-23R and the p40 subunit binds to IL-12RB1.

The anti-IL-23 antibody may be an anti-IL-23p19 antibody. The anti-IL-23p19 antibody may refer to any antibody that specifically binds to the p19 subunit of IL-23.

The anti-IL-23 antibody may be guselkumab (CAS No. 1350289-85-8). Accordingly, the liquid formulation may be a stable liquid formulation of guselkumab. Guselkumab is a human immunoglobulin G1 (human IgG1) monoclonal antibody that binds to the p19 subunit of IL-23. Guselkumab is commercially available under the trade name Tremfya^{®}. Tremfya^{®} has been approved by the U.S. Food and Drug Administration (FDA) as a therapeutic agent for plaque psoriasis.Subsequently, it has received additional FDA approvals for the treatment of palmoplantar pustulosis, psoriatic arthritis, and the like. Most recently, it has also received U.S. FDA approval as a therapeutic agent for ulcerative colitis. The sequence of guselkumab is known and may be produced by general methods known in the art. More detailed information on guselkumab can be readily obtained by those skilled in the art from publicly available databases.

The term "guselkumab" as used herein may also be interpreted to comprise guselkumab variants in which the amino acid sequence is modified (deleted, inserted, and/or substituted) and/or the glycosylation characteristics are modified, provided that such modifications do not affect the polypeptide function.

The anti-IL-23 antibody may be comprised in the liquid formulation in a therapeutically effective amount.

The concentration of the anti-IL-23 antibody may be about 1 mg/mL to about 500 mg/mL, about 1 mg/mL to about 400 mg/mL, about 1 mg/mL to about 300 mg/mL, about 1 mg/mL to about 250 mg/mL, about 1 mg/mL to about 200 mg/mL, about 1 mg/mL to about 175 mg/mL, about 1 mg/mL to about 150 mg/mL, about 1 mg/mL to about 125 mg/mL, about 1 mg/mL to about 100 mg/mL, about 1 mg/mL to about 75 mg/mL, about 1 mg/mL to about 50 mg/mL, about 1 mg/mL to about 30 mg/mL, about 1 mg/mL to about 20 mg/mL, about 1 mg/mL to about 10 mg/mL, about 2 mg/mL to about 500 mg/mL, about 2 mg/mL to about 400 mg/mL, about 2 mg/mL to about 300 mg/mL, about 2 mg/mL to about 250 mg/mL, about 2 mg/mL to about 200 mg/mL, about 2 mg/mL to about 175 mg/mL, about 2 mg/mL to about 150 mg/mL, about 2 mg/mL to about 125 mg/mL, about 2 mg/mL to about 100 mg/mL, about 2 mg/mL to about 75 mg/mL, about 2 mg/mL to about 50 mg/mL, about 2 mg/mL to about 30 mg/mL, about 2 mg/mL to about 20 mg/mL, about 2 mg/mL to about 10 mg/mL, about 5 mg/mL to about 500 mg/mL, about 5 mg/mL to about 400 mg/mL, about 5 mg/mL to about 300 mg/mL, about 5 mg/mL to about 250 mg/mL, about 5 mg/mL to about 200 mg/mL, about 5 mg/mL to about 175 mg/mL, about 5 mg/mL to about 150 mg/mL, about 5 mg/mL to about 125 mg/mL, about 5 mg/mL to about 100 mg/mL, about 5 mg/mL to about 75 mg/mL, about 5 mg/mL to about 50 mg/mL, about 5 mg/mL to about 30 mg/mL, about 5 mg/mL to about 20 mg/mL, about 5 mg/mL to about 10 mg/mL, about 10 mg/mL to about 500 mg/mL, about 10 mg/mL to about 400 mg/mL, about 10 mg/mL to about 300 mg/mL, about 10 mg/mL to about 250 mg/mL, about 10 mg/mL to about 200 mg/mL, about 10 mg/mL to about 175 mg/mL, about 10 mg/mL to about 150 mg/mL, about 10 mg/mL to about 125 mg/mL, about 10 mg/mL to about 100 mg/mL, about 10 mg/mL to about 75 mg/mL, about 10 mg/mL to about 50 mg/mL, about 10 mg/mL to about 30 mg/mL, about 10 mg/mL to about 20 mg/mL, about 25 mg/mL to about 500 mg/mL, about 25 mg/mL to about 400 mg/mL, about 25 mg/mL to about 300 mg/mL, about 25 mg/mL to about 250 mg/mL, about 25 mg/mL to about 200 mg/mL, about 25 mg/mL to about 175 mg/mL, about 25 mg/mL to about 150 mg/mL, about 25 mg/mL to about 125 mg/mL, about 25 mg/mL to about 100 mg/mL, about 25 mg/mL to about 75 mg/mL, about 25 mg/mL to about 50 mg/mL, about 25 mg/mL to about 30 mg/mL, about 50 mg/mL to about 500 mg/mL, about 50 mg/mL to about 400 mg/mL, about 50 mg/mL to about 300 mg/mL, about 50 mg/mL to about 250 mg/mL, about 50 mg/mL to about 200 mg/mL, about 50 mg/mL to about 175 mg/mL, about 50 mg/mL to about 150 mg/mL, about 50 mg/mL to about 125 mg/mL, about 50 mg/mL to about 100 mg/mL, about 50 mg/mL to about 75 mg/mL, about 75 mg/mL to about 500 mg/mL, about 75 mg/mL to about 400 mg/mL, about 75 mg/mL to about 300 mg/mL, about 75 mg/mL to about 250 mg/mL, about 75 mg/mL to about 200 mg/mL, about 75 mg/mL to about 175 mg/mL, about 75 mg/mL to about 150 mg/mL, about 75 mg/mL to about 125 mg/mL, about 75 mg/mL to about 100 mg/mL, about 90 mg/mL to about 500 mg/mL, about 90 mg/mL to about 400 mg/mL, about 90 mg/mL to about 300 mg/mL, about 90 mg/mL to about 250 mg/mL, about 90 mg/mL to about 200 mg/mL, about 90 mg/mL to about 175 mg/mL, about 90 mg/mL to about 150 mg/mL, about 90 mg/mL to about 125 mg/mL, about 90 mg/mL to about 110 mg/mL, about 90 mg/mL to about 100 mg/mL, about 100 mg/mL to about 500 mg/mL, about 100 mg/mL to about 400 mg/mL, about 100 mg/mL to about 300 mg/mL, about 100 mg/mL to about 250 mg/mL, about 100 mg/mL to about 200 mg/mL, about 100 mg/mL to about 175 mg/mL, about 100 mg/mL to about 150 mg/mL, about 100 mg/mL to about 125 mg/mL, or about 100 mg/mL to about 110 mg/mL.

In an embodiment, the concentration of the anti-IL-23 antibody may be about 2 mg/ml, about 4 mg/ml, about 5 mg/ml, about 6 mg/ml, about 8 mg/ml, about 10 mg/ml, about 20 mg/ml, about 25 mg/ml, about 50 mg/ml, about 75 mg/ml, about 100 mg/ml, about 125 mg/ml, about 150 mg/ml, about 175 mg/ml, about 200 mg/ml, about 250 mg/ml, or about 300 mg/ml.

In certain embodiments, the concentration of the anti-IL-23 antibody may be about 100 mg/ml.

### (2) Buffer and pH

A liquid formulation according to an aspect comprises a buffer. The buffer may function to adjust the pH of the formulation. The pH of the liquid formulation may be maintained at a certain value or within a certain range by the buffer. Therefore, the buffer may serve to provide the liquid formulation with a pH of a certain value or range.

The buffer may be used without limitation as to its type, provided that it is applicable to biopharmaceuticals.

The buffer may comprise at least one selected from acetate, histidine, phosphate, citrate, succinate, malate, tartarate, carbonate, salts thereof, and hydrates thereof.

The term "salt" may be any pharmaceutically acceptable salt. The salt may comprise inorganic acid salts, organic acid salts, metal salts, etc. of the compound. The inorganic acid salts may be hydrochloride, hydrobromide, phosphate, sulfate, or disulfate. The organic acid salts may be formate, acetate, propionate, lactate, oxalate, tartrate, malate, maleate, citrate, fumarate, besylate, camsylate, edicylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, sodium salt, magnesium salt, strontium salt, or potassium salt.

The term "hydrate" refers to a substance containing water molecules within its molecular structure. The hydrate may be a monohydrate, a dihydrate, or a trihydrate. For example, a hydrate of histidine may comprise histidine monohydrochloride monohydrate.

The pH of the liquid formulation according to an aspect may be about 4.0 to about 8.5. The pH of the liquid formulation may be provided by the buffer.

Specifically, the pH of the formulation may be any range or any value selected about 4.0 to about 8.5. For example, the pH may be about 4.0 to about 8.5, about 4.0 to about 8.0, about 4.0 to about 7.5, about 4.0 to about 7.2, about 4.0 to about 7.0, about 4.0 to about 6.9, about 4.0 to about 6.8, about 4.0 to about 6.7, about 4.0 to about 6.6, about 4.0 to about 6.5, about 4.0 to about 6.4, about 4.0 to about 6.3, about 4.0 to about 6.2, about 4.0 to about 6.1, about 4.0 to about 6.0, about 4.0 to about 5.9, about 4.0 to about 5.8, about 4.0 to about 5.7, about 4.0 to about 5.6, about 4.0 to about 5.5, about 4.0 to about 5.2, about 4.0 to about 5.0, about 4.0 to about 4.7, about 4.0 to about 4.5, about 4.5 to about 8.5, about 4.5 to about 8.0, about 4.5 to about 7.5, about 4.5 to about 7.2, about 4.5 to about 7.0, about 4.5 to about 6.9, about 4.5 to about 6.8, about 4.5 to about 6.7, about 4.5 to about 6.6, about 4.5 to about 6.5, about 4.5 to about 6.4, about 4.5 to about 6.3, about 4.5 to about 6.2, about 4.5 to about 6.1, about 4.5 to about 6.0, about 4.5 to about 5.9, about 4.5 to about 5.8, about 4.5 to about 5.7, about 4.5 to about 5.6, about 4.5 to about 5.5, about 4.5 to about 5.2, about 4.5 to about 5.0, about 4.5 to about 4.7, about 4.8 to about 8.5, about 4.8 to about 8.0, about 4.8 to about 7.5, about 4.8 to about 7.2, about 4.8 to about 7.0, about 4.8 to about 6.9, about 4.8 to about 6.8, about 4.8 to about 6.7, about 4.8 to about 6.6, about 4.8 to about 6.5, about 4.8 to about 6.4, about 4.8 to about 6.3, about 4.8 to about 6.2, about 4.8 to about 6.1, about 4.8 to about 6.0, about 4.8 to about 5.9, about 4.8 to about 5.8, about 4.8 to about 5.7, about 4.8 to about 5.6, about 4.8 to about 5.5, about 4.8 to about 5.2, about 4.8 to about 5.0, about 5.0 to about 8.5, about 5.0 to about 8.0, about 5.0 to about 7.5, about 5.0 to about 7.2, about 5.0 to about 7.0, about 5.0 to about 6.9, about 5.0 to about 6.8, about 5.0 to about 6.7, about 5.0 to about 6.6, about 5.0 to about 6.5, about 5.0 to about 6.4, about 5.0 to about 6.3, about 5.0 to about 6.2, about 5.0 to about 6.1, about 5.0 to about 6.0, about 5.0 to about 5.9, about 5.0 to about 5.8, about 5.0 to about 5.7, about 5.0 to about 5.6, about 5.0 to about 5.5, about 5.0 to about 5.2, about 5.3 to about 8.5, about 5.3 to about 8.0, about 5.3 to about 7.5, about 5.3 to about 7.2, about 5.3 to about 7.0, about 5.3 to about 6.9, about 5.3 to about 6.8, about 5.3 to about 6.7, about 5.3 to about 6.6, about 5.3 to about 6.5, about 5.3 to about 6.4, about 5.3 to about 6.3, about 5.3 to about 6.2, about 5.3 to about 6.1, about 5.3 to about 6.0, about 5.3 to about 5.9, about 5.3 to about 5.8, about 5.3 to about 5.7, about 5.3 to about 5.6, about 5.3 to about 5.5, about 5.5 to about 8.5, about 5.5 to about 8.0, about 5.5 to about 7.5, about 5.5 to about 7.2, about 5.5 to about 7.0, about 5.5 to about 6.9, about 5.5 to about 6.8, about 5.5 to about 6.7, about 5.5 to about 6.6, about 5.5 to about 6.5, about 5.5 to about 6.4, about 5.5 to about 6.3, about 5.5 to about 6.2, about 5.5 to about 6.1, about 5.5 to about 6.0, about 5.5 to about 5.9, about 5.5 to about 5.8, about 5.5 to about 5.7, about 5.6 to about 8.5, about 5.6 to about 8.0, about 5.6 to about 7.5, about 5.6 to about 7.2, about 5.6 to about 7.0, about 5.6 to about 6.9, about 5.6 to about 6.8, about 5.6 to about 6.7, about 5.6 to about 6.6, about 5.6 to about 6.5, about 5.6 to about 6.4, about 5.6 to about 6.3, about 5.6 to about 6.2, about 5.6 to about 6.1, about 5.6 to about 6.0, about 5.6 to about 5.9, about 5.6 to about 5.8, about 5.6 to about 5.7, about 5.8 to about 8.5, about 5.8 to about 8.2, about 5.8 to about 8.0, about 5.8 to about 7.5, about 5.8 to about 7.2, about 5.8 to about 7.0, about 5.8 to about 6.9, about 5.8 to about 6.8, about 5.8 to about 6.7, about 5.8 to about 6.6, about 5.8 to about 6.5, about 5.8 to about 6.4, about 5.8 to about 6.3, about 5.8 to about 6.2, about 5.8 to about 6.1, about 5.8 to about 6.0, about 5.9 to about 8.5, about 5.9 to about 8.2, about 5.9 to about 8.0, about 5.9 to about 7.7, about 5.9 to about 7.5, about 5.9 to about 7.2, about 5.9 to about 7.0, about 5.9 to about 6.9, about 5.9 to about 6.8, about 5.9 to about 6.7, about 5.9 to about 6.6, about 5.9 to about 6.5, about 5.9 to about 6.4, about 5.9 to about 6.3, about 5.9 to about 6.2, about 5.9 to about 6.1, about 5.9 to about 6.0, about 6.0 to about 8.5, about 6.0 to about 8.2, about 6.0 to about 8.0, about 6.0 to about 7.7, about 6.0 to about 7.5, about 6.0 to about 7.2, about 6.0 to about 7.0, about 6.0 to about 6.9, about 6.0 to about 6.8, about 6.0 to about 6.7, about 6.0 to about 6.6, about 6.0 to about 6.5, about 6.0 to about 6.4, about 6.0 to about 6.3, about 6.0 to about 6.2, about 6.0 to about 6.1, about 6.3 to about 8.5, about 6.3 to about 8.2, about 6.3 to about 8.0, about 6.3 to about 7.7, about 6.3 to about 7.5, about 6.3 to about 7.2, about 6.3 to about 7.0, about 6.3 to about 6.9, about 6.3 to about 6.8, about 6.3 to about 6.7, about 6.3 to about 6.6, about 6.3 to about 6.5, about 6.5 to about 8.5, about 6.5 to about 8.2, about 6.5 to about 8.0, about 6.5 to about 7.7, about 6.5 to about 7.5, about 6.5 to about 7.2, about 6.5 to about 7.0, about 6.5 to about 6.9, about 6.5 to about 6.8, about 6.5 to about 6.7, about 7.0 to about 8.5, about 7.0 to about 8.2, about 7.0 to about 8.0, about 7.0 to about 7.7, about 7.0 to about 7.5, or about 7.0 to about 7.2.

In an embodiment, the pH of the liquid formulation may be about 4.0, about 4.5, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0.

In certain embodiments, the buffer may comprise one or more selected from acetate, histidine, phosphate, citrate, succinate, salts thereof, and hydrates thereof.

In certain embodiments, the buffer may comprise one or more selected from acetate, histidine, phosphate, salts thereof, and hydrates thereof. In such embodiments, the pH of the liquid formulation may be about 4.0 to about 8.5, about 4.0 to about 8.0, about 4.0 to about 6.0, about 5.0 to about 7.0, about 5.5 to about 8.0, about 6.0 to about 8.0, about 5.9 to about 8.5, about 5.9 to about 8.0, about 5.9 to about 7.5, about 5.9 to about 7.0, about 5.9 to about 6.5, about 5.9 to about 6.2, about 5.9 to about 6.1, about 5.9 to about 6.0, or about 6.0, or about 5.8.

In certain embodiments, the buffer may comprise one or more selected from acetate, a salt thereof, and a hydrate thereof. In such embodiments, the pH of the liquid formulation may be any range or any value selected from the range of about 4.0 to about 6.5, about 4.0 to about 6.2, about 4.0 to about 6.0, about 4.0 to about 5.2, or about 4.0 to about 5.0, and for example, the pH may be 4.0, 4.5, 5.0, 5.5, 5.8, or 6.0.

In certain embodiments, the buffer may comprise one or more selected from histidine, a salt thereof, and a hydrate thereof. In such embodiments, the pH of the liquid formulation may be any range or any value selected from the range of about 4.5 to about 7.5, about 4.8 to about 7.2, about 5.0 to about 7.0, about 5.6 to about 7.5, about 5.6 to about 7.2, about 5.6 to about 7.0, about 5.9 to about 7.5, about 5.9 to about 7.2, about 5.9 to about 7.0, about 6.0 to about 7.5, about 6.0 to about 7.2, or about 6.0 to about 7.0, for example, the pH may be 5.0, 5.5, 5.8, 6.0, 6.5, or 7.0.

In certain embodiments, the buffer may comprise one or more selected from phosphate, a salt thereof, and a hydrate thereof. In such embodiments, the pH of the liquid formulation may be any range or any value selected from the range of about 5.5 to about 8.5, about 5.8 to about 8.2, about 5.8 to about 6.7, about 5.9 to about 8.2, about 5.9 to about 8.0, or about 6.0 to about 8.0, for example, the pH may be 5.8, 6.0, 6.5, 7.0, 7.5, or 8.0.

In certain embodiments, the buffer may comprise one or more selected from citrate, a salt thereof, and a hydrate thereof. In such embodiments, the pH of the liquid formulation may be any range or any value selected from the range of about 4.5 to about 7.0, about 5.3 to about 7.0, about 5.3 to about 6.7, about 5.5 to about 7.0, about 5.5 to about 6.7, about 5.5 to about 6.5, about 5.8 to about 6.7, about 5.9 to about 6.7, about 5.9 to about 6.5, or about 6.0 to about 6.5, for example, the pH may be 5.5, 5.8, 6.0, or 6.5.

In certain embodiments, the buffer may comprise one or more selected from succinate, a salt thereof, and a hydrate thereof. In such embodiments, the pH of the liquid formulation may be any range or any value selected from the range of about 4.3 to about 6.5, about 4.3 to about 6.2, about 4.3 to about 6.0, about 4.5 to about 6.5, about 4.5 to about 6.2, or about 4.5 to 6.0, for example, the pH may be 4.5, 5.0, 5.5, 5.8, or 6.0.

In certain embodiments, a combination of the buffer and pH may be selected from the following, and specifically, the liquid formulation may have an aggregation temperature (T_{agg}) of about 73.83 °C. or higher:
A) the buffer comprises acetate, and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

In certain embodiments, a combination of the buffer and pH may be selected from the following, and specifically, the liquid formulation may have a diffusion interaction parameter (K_{D}) of about 20.0 mL/g or higher:
A) the buffer comprises acetate and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

The concentration of the buffer may be any range or any value selected about 0.1 mM to about 150 mM. For example, the concentration of the buffer may be about 0.1 mM to about 150 mM, about 0.1 mM to about 120 mM, about 0.1 mM to about 100 mM, about 0.1 mM to about 80 mM, about 0.1 mM to about 70 mM, about 0.1 mM to about 50 mM, about 0.1 mM to about 40 mM, about 0.1 mM to about 30 mM, about 0.1 mM to about 20 mM, about 0.1 mM to about 15 mM, about 0.1 mM to about 12 mM, about 0.1 mM to about 10 mM, about 0.1 mM to about 9 mM, about 0.1 mM to about 8 mM, about 0.1 mM to about 7 mM, about 0.1 mM to about 6 mM, about 0.1 mM to about 5 mM, about 0.1 mM to about 4 mM, about 0.1 mM to about 3 mM, about 0.1 mM to about 2 mM, about 0.5 mM to about 150 mM, about 0.5 mM to about 120 mM, about 0.5 mM to about 100 mM, about 0.5 mM to about 80 mM, about 0.5 mM to about 70 mM, about 0.5 mM to about 50 mM, about 0.5 mM to about 40 mM, about 0.5 mM to about 30 mM, about 0.5 mM to about 20 mM, about 0.5 mM to about 15 mM, about 0.5 mM to about 12 mM, about 0.5 mM to about 10 mM, about 0.5 mM to about 9 mM, about 0.5 mM to about 8 mM, about 0.5 mM to about 7 mM, about 0.5 mM to about 6 mM, about 0.5 mM to about 5 mM, about 0.5 mM to about 4 mM, about 0.5 mM to about 3 mM, about 0.5 mM to about 2 mM, about 1 mM to about 150 mM, about 1 mM to about 120 mM, about 1 mM to about 100 mM, about 1 mM to about 80 mM, about 1 mM to about 70 mM, about 1 mM to about 50 mM, about 1 mM to about 40 mM, about 1 mM to about 30 mM, about 1 mM to about 20 mM, about 1 mM to about 15 mM, about 1 mM to about 12 mM, about 1 mM to about 10 mM, about 1 mM to about 9 mM, about 1 mM to about 8 mM, about 1 mM to about 7 mM, about 1 mM to about 6 mM, about 1 mM to about 5 mM, about 1 mM to about 4 mM, about 1 mM to about 3 mM, about 1 mM to about 2 mM, about 5 mM to about 150 mM, about 5 mM to about 120 mM, about 5 mM to about 100 mM, about 5 mM to about 80 mM, about 5 mM to about 70 mM, about 5 mM to about 50 mM, about 5 mM to about 40 mM, about 5 mM to about 30 mM, about 5 mM to about 20 mM, about 5 mM to about 19 mM, about 5 mM to about 18 mM, about 5 mM to about 17 mM, about 5 mM to about 16 mM, about 5 mM to about 15 mM, about 5 mM to about 14 mM, about 5 mM to about 13 mM, about 5 mM to about 12 mM, about 5 mM to about 11 mM, about 5 mM to about 10 mM, about 5 mM to about 9 mM, about 5 mM to about 8 mM, about 5 mM to about 7 mM, about 5 mM to about 6 mM, about 6 mM to about 150 mM, about 6 mM to about 120 mM, about 6 mM to about 100 mM, about 6 mM to about 80 mM, about 6 mM to about 70 mM, about 6 mM to about 50 mM, about 6 mM to about 40 mM, about 6 mM to about 30 mM, about 6 mM to about 20 mM, about 6 mM to about 19 mM, about 6 mM to about 18 mM, about 6 mM to about 17 mM, about 6 mM to about 16 mM, about 6 mM to about 15 mM, about 6 mM to about 14 mM, about 6 mM to about 13 mM, about 6 mM to about 12 mM, about 6 mM to about 11 mM, about 6 mM to about 10 mM, about 6 mM to about 9 mM, about 6 mM to about 8 mM, about 6 mM to about 7 mM, about 7 mM to about 150 mM, about 7 mM to about 120 mM, about 7 mM to about 100 mM, about 7 mM to about 80 mM, about 7 mM to about 70 mM, about 7 mM to about 50 mM, about 7 mM to about 40 mM, about 7 mM to about 30 mM, about 7 mM to about 20 mM, about 7 mM to about 19 mM, about 7 mM to about 18 mM, about 7 mM to about 17 mM, about 7 mM to about 16 mM, about 7 mM to about 15 mM, about 7 mM to about 14 mM, about 7 mM to about 13 mM, about 7 mM to about 12 mM, about 7 mM to about 11 mM, about 7 mM to about 10 mM, about 7 mM to about 9 mM, about 7 mM to about 8 mM, about 8 mM to about 150 mM, about 8 mM to about 120 mM, about 8 mM to about 100 mM, about 8 mM to about 80 mM, about 8 mM to about 70 mM, about 8 mM to about 50 mM, about 8 mM to about 40 mM, about 8 mM to about 30 mM, about 8 mM to about 20 mM, about 8 mM to about 19 mM, about 8 mM to about 18 mM, about 8 mM to about 17 mM, about 8 mM to about 16 mM, about 8 mM to about 15 mM, about 8 mM to about 14 mM, about 8 mM to about 13 mM, about 8 mM to about 12 mM, about 8 mM to about 11 mM, about 8 mM to about 10 mM, about 8 mM to about 9 mM, about 9 mM to about 150 mM, about 9 mM to about 120 mM, about 9 mM to about 100 mM, about 9 mM to about 80 mM, about 9 mM to about 70 mM, about 9 mM to about 50 mM, about 9 mM to about 40 mM, about 9 mM to about 30 mM, about 9 mM to about 20 mM, about 9 mM to about 19 mM, about 9 mM to about 18 mM, about 9 mM to about 17 mM, about 9 mM to about 16 mM, about 9 mM to about 15 mM, about 9 mM to about 14 mM, about 9 mM to about 13 mM, about 9 mM to about 12 mM, about 9 mM to about 11 mM, about 9 mM to about 10 mM, about 10 mM to about 150 mM, about 10 mM to about 120 mM, about 10 mM to about 100 mM, about 10 mM to about 80 mM, about 10 mM to about 70 mM, about 10 mM to about 50 mM, about 10 mM to about 40 mM, about 10 mM to about 30 mM, about 10 mM to about 20 mM, about 10 mM to about 19 mM, about 10 mM to about 18 mM, about 10 mM to about 17 mM, about 10 mM to about 16 mM, about 10 mM to about 15 mM, about 10 mM to about 14 mM, about 10 mM to about 13 mM, about 10 mM to about 12 mM, about 10 mM to about 11 mM, about 15 mM to about 150 mM, about 15 mM to about 120 mM, about 15 mM to about 100 mM, about 15 mM to about 80 mM, about 15 mM to about 70 mM, about 15 mM to about 50 mM, about 15 mM to about 40 mM, about 15 mM to about 30 mM, about 15 mM to about 25 mM, about 15 mM to about 22 mM, about 18 mM to about 150 mM, about 18 mM to about 120 mM, about 18 mM to about 100 mM, about 18 mM to about 80 mM, about 18 mM to about 70 mM, about 18 mM to about 50 mM, about 18 mM to about 40 mM, about 18 mM to about 30 mM, about 18 mM to about 25 mM, about 18 mM to about 22 mM, or about 19 mM to about 21 mM.

In an embodiment, the concentration of the buffer may be any range or any value selected from the range of about 11 mM to about 70 mM, about 11 mM to about 50 mM, or about 15 mM to about 40 mM.

In certain embodiments, the concentration of the buffer may be about 20 mM, about 30 mM, about 40 mM, about 50 mM, about 60 mM, about 70 mM, about 80 mM, about 90 mM, about 100 mM, about 120 mM, or about 150 mM.

### (3) Stabilizer

A liquid formulation according to an aspect may comprise a stabilizer.

The term "stabilizer" refers to a substance that is added to prevent physical or chemical changes when preserving a substance.

The stabilizer may be used without limitation as to its type, provided that it is applicable to biopharmaceuticals.

The stabilizer may comprise at least one selected from sugars, sugar alcohols, amino acids, metal salts, salts thereof, and hydrates thereof.

The sugar may be a monosaccharide, a disaccharide, an oligosaccharide, or a polysaccharide. The sugar may comprise one or more selected from trehalose, sucrose, galactose, mannose, maltose, lactose, fructose, and glucose.

The sugar alcohol is a general term for polyols having two or more hydroxyl groups, which is obtained by reducing an aldehyde group or a ketone group of a sugar to an alcohol group. The sugar alcohol may comprise at least one selected from mannitol, sorbitol, xylitol, arabitol, erythritol, lactitol, maltitol, and inositol. The sugar alcohol comprises anhydrides or hydrates of the sugar alcohol. For example, trehalose may comprise not only trehalose but also trehalose dihydrate.

The amino acids may comprise one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

The metal salt may comprise one or more selected from NaCl, KCl, NaF, KBr, NaBr, Na₂SO₄, NaSCN, CaCl₂, MgCl₂, and K₂SO₄.

The concentration of the sugar may be freely adjusted within a range that maintains the stability of the antibody, and may vary individually depending on each specific sugar type. The concentration of the sugar may be any range or any value selected from within the range of about 0.1% (w/v) to about 20.0% (w/v). For example, the concentration of the sugar may be about 0.1% (w/v) to about 20.0% (w/v), about 0.1% (w/v) to about 15.0% (w/v), about 0.1% (w/v) to about 12.0% (w/v), about 0.1% (w/v) to about 10.0% (w/v), about 0.1% (w/v) to about 8.0% (w/v), about 0.1% (w/v) to about 5.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 1.0% (w/v) to about 8.0% (w/v), about 1.0% (w/v) to about 5.0% (w/v), about 2.0% (w/v) to about 20.0% (w/v), about 2.0% (w/v) to about 15.0% (w/v), about 2.0% (w/v) to about 12.0% (w/v), about 2.0% (w/v) to about 10.0% (w/v), about 2.0% (w/v) to about 8.0% (w/v), about 2.0% (w/v) to about 5.0% (w/v), about 4.0% (w/v) to about 20.0% (w/v), about 4.0% (w/v) to about 15.0% (w/v), about 4.0% (w/v) to about 12.0% (w/v), about 4.0% (w/v) to about 10.0% (w/v), about 4.0% (w/v) to about 9.0% (w/v), about 4.0% (w/v) to about 8.0% (w/v), about 4.0% (w/v) to about 5.0% (w/v), about 5.0% (w/v) to about 20.0% (w/v), about 5.0% (w/v) to about 15.0% (w/v), about 5.0% (w/v) to about 12.0% (w/v), about 5.0% (w/v) to about 10.0% (w/v), about 5.0% (w/v) to about 9.0% (w/v), about 5.0% (w/v) to about 8.0% (w/v), about 6.0% (w/v) to about 20.0% (w/v), about 6.0% (w/v) to about 15.0% (w/v), about 6.0% (w/v) to about 12.0% (w/v), about 6.0% (w/v) to about 10.0% (w/v), about 6.0% (w/v) to about 9.0% (w/v), about 6.0% (w/v) to about 8.0% (w/v), about 7.0% (w/v) to about 20.0% (w/v), about 7.0% (w/v) to about 15.0% (w/v), about 7.0% (w/v) to about 12.0% (w/v), about 7.0% (w/v) to about 10.0% (w/v), about 7.0% (w/v) to about 9.0% (w/v), about 7.0% (w/v) to about 8.0% (w/v), about 7.5% (w/v) to about 20.0% (w/v), about 7.5% (w/v) to about 15.0% (w/v), about 7.5% (w/v) to about 12.0% (w/v), about 7.5% (w/v) to about 10.0% (w/v), about 7.5% (w/v) to about 9.0% (w/v), about 7.5% (w/v) to about 8.5% (w/v), about 7.5% (w/v) to about 8.0% (w/v), about 8.0% (w/v) to about 20.0% (w/v), about 8.0% (w/v) to about 15.0% (w/v), about 8.0% (w/v) to about 14.0% (w/v), about 8.0% (w/v) to about 13.0% (w/v), about 8.0% (w/v) to about 12.0% (w/v), about 8.0% (w/v) to about 11.0% (w/v), about 8.0% (w/v) to about 10.0% (w/v), about 8.0% (w/v) to about 9.5% (w/v), about 8.0% (w/v) to about 9.0% (w/v), about 8.5% (w/v) to about 20.0% (w/v), about 8.5% (w/v) to about 15.0% (w/v), about 8.5% (w/v) to about 14.0% (w/v), about 8.5% (w/v) to about 13.0% (w/v), about 8.5% (w/v) to about 12.0% (w/v), about 8.5% (w/v) to about 11.0% (w/v), about 8.5% (w/v) to about 10.0% (w/v), or about 8.5% (w/v) to about 9.5% (w/v).

The concentration of the sugar alcohol may be freely adjusted within a range that maintains the stability of the antibody, and may vary individually depending on each specific sugar alcohol type. The concentration of the sugar alcohol may be any range or any value selected about 0.1% (w/v) to about 20.0% (w/v). For example, the concentration of the sugar alcohol may be about 0.1% (w/v) to about 20.0% (w/v), about 0.1% (w/v) to about 15.0% (w/v), about 0.1% (w/v) to about 12.0% (w/v), about 0.1% (w/v) to about 10.0% (w/v), about 0.1% (w/v) to about 8.0% (w/v), about 0.1% (w/v) to about 5.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 1.0% (w/v) to about 8.0% (w/v), about 1.0% (w/v) to about 5.0% (w/v), about 2.0% (w/v) to about 20.0% (w/v), about 2.0% (w/v) to about 15.0% (w/v), about 2.0% (w/v) to about 12.0% (w/v), about 2.0% (w/v) to about 10.0% (w/v), about 2.0% (w/v) to about 8.0% (w/v), about 2.0% (w/v) to about 5.0% (w/v), about 3.0% (w/v) to about 20.0% (w/v), about 3.0% (w/v) to about 15.0% (w/v), about 3.0% (w/v) to about 12.0% (w/v), about 3.0% (w/v) to about 10.0% (w/v), about 3.0% (w/v) to about 9.0% (w/v), about 3.0% (w/v) to about 8.0% (w/v), about 3.0% (w/v) to about 7.0% (w/v), about 3.0% (w/v) to about 6.0% (w/v), about 3.0% (w/v) to about 5.0% (w/v), about 3.0% (w/v) to about 4.5% (w/v), about 3.0% (w/v) to about 4.0% (w/v), about 3.0% (w/v) to about 3.5% (w/v), about 4.0% (w/v) to about 20.0% (w/v), about 4.0% (w/v) to about 15.0% (w/v), about 4.0% (w/v) to about 12.0% (w/v), about 4.0% (w/v) to about 10.0% (w/v), about 4.0% (w/v) to about 9.0% (w/v), about 4.0% (w/v) to about 8.0% (w/v), about 4.0% (w/v) to about 7.0% (w/v), about 4.0% (w/v) to about 6.0% (w/v), about 4.0% (w/v) to about 5.0% (w/v), about 4.5% (w/v) to about 20.0% (w/v), about 4.5% (w/v) to about 15.0% (w/v), about 4.5% (w/v) to about 12.0% (w/v), about 4.5% (w/v) to about 10.0% (w/v), about 4.5% (w/v) to about 9.0% (w/v), about 4.5% (w/v) to about 8.0% (w/v), about 4.5% (w/v) to about 7.0% (w/v), about 4.5% (w/v) to about 6.0% (w/v), or about 4.5% (w/v) to about 5.0% (w/v).

The concentration of the amino acids may be freely adjusted within a range that maintains the stability of the antibody, and may vary individually depending on each specific amino acid type. The concentration of the amino acid may be any range or any value selected about 1 mM to about 400 mM. For example, the concentration of the amino acid may be about 1 mM to about 400 mM, about 1 mM to about 300 mM, about 1 mM to about 200 mM, about 1 mM to about 100 mM, about 10 mM to about 400 mM, about 10 mM to about 380 mM, about 10 mM to about 350 mM, about 10 mM to about 310 mM, about 10 mM to about 300 mM, about 10 mM to about 290 mM, about 10 mM to about 260 mM, about 10 mM to about 200 mM, about 10 mM to about 180 mM, about 10 mM to about 160 mM, about 10 mM to about 100 mM, about 20 mM to about 400 mM, about 20 mM to about 380 mM, about 20 mM to about 350 mM, about 20 mM to about 310 mM, about 20 mM to about 300 mM, about 20 mM to about 290 mM, about 20 mM to about 260 mM, about 20 mM to about 200 mM, about 20 mM to about 180 mM, about 20 mM to about 160 mM, about 20 mM to about 150 mM, about 20 mM to about 100 mM, about 20 mM to about 50 mM, about 20 mM to about 40 mM, about 20 mM to about 30 mM, about 30 mM to about 400 mM, about 30 mM to about 380 mM, about 30 mM to about 350 mM, about 30 mM to about 310 mM, about 30 mM to about 300 mM, about 30 mM to about 290 mM, about 30 mM to about 260 mM, about 30 mM to about 200 mM, about 30 mM to about 180 mM, about 30 mM to about 160 mM, about 30 mM to about 150 mM, about 30 mM to about 100 mM, about 30 mM to about 80 mM, about 30 mM to about 50 mM, about 30 mM to about 40 mM, about 40 mM to about 400 mM, about 40 mM to about 380 mM, about 40 mM to about 350 mM, about 40 mM to about 310 mM, about 40 mM to about 300 mM, about 40 mM to about 290 mM, about 40 mM to about 260 mM, about 40 mM to about 200 mM, about 40 mM to about 180 mM, about 40 mM to about 160 mM, about 40 mM to about 150 mM, about 40 mM to about 100 mM, about 40 mM to about 80 mM, about 40 mM to about 50 mM, about 50 mM to about 400 mM, about 50 mM to about 380 mM, about 50 mM to about 350 mM, about 50 mM to about 310 mM, about 50 mM to about 300 mM, about 50 mM to about 290 mM, about 50 mM to about 260 mM, about 50 mM to about 250 mM, about 50 mM to about 180 mM, about 50 mM to about 160 mM, about 50 mM to about 200 mM, about 50 mM to about 150 mM, about 50 mM to about 100 mM, about 50 mM to about 80 mM, about 60 mM to about 400 mM, about 60 mM to about 380 mM, about 60 mM to about 350 mM, about 60 mM to about 310 mM, about 60 mM to about 300 mM, about 60 mM to about 250 mM, about 60 mM to about 200 mM, about 60 mM to about 150 mM, about 60 mM to about 100 mM, about 60 mM to about 80 mM, about 70 mM to about 400 mM, about 70 mM to about 380 mM, about 70 mM to about 350 mM, about 70 mM to about 310 mM, about 70 mM to about 300 mM, about 70 mM to about 250 mM, about 70 mM to about 200 mM, about 70 mM to about 150 mM, about 70 mM to about 100 mM, about 70 mM to about 90 mM, about 70 mM to about 80 mM, about 80 mM to about 400 mM, about 80 mM to about 380 mM, about 80 mM to about 350 mM, about 80 mM to about 310 mM, about 80 mM to about 300 mM, about 80 mM to about 250 mM, about 80 mM to about 200 mM, about 80 mM to about 150 mM, about 80 mM to about 120 mM, about 80 mM to about 100 mM, about 90 mM to about 400 mM, about 90 mM to about 380 mM, about 90 mM to about 350 mM, about 90 mM to about 310 mM, about 90 mM to about 300 mM, about 90 mM to about 250 mM, about 90 mM to about 200 mM, about 90 mM to about 150 mM, about 90 mM to about 100 mM, about 100 mM to about 400 mM, about 100 mM to about 380 mM, about 100 mM to about 350 mM, about 100 mM to about 310 mM, about 100 mM to about 300 mM, about 100 mM to about 280 mM, about 100 mM to about 250 mM, about 100 mM to about 220 mM, about 100 mM to about 200 mM, about 100 mM to about 180 mM, about 100 mM to about 150 mM, about 110 mM to about 400 mM, about 110 mM to about 300 mM, about 110 mM to about 280 mM, about 110 mM to about 250 mM, about 110 mM to about 220 mM, about 110 mM to about 200 mM, about 110 mM to about 180 mM, about 110 mM to about 150 mM, about 120 mM to about 400 mM, about 120 mM to about 300 mM, about 120 mM to about 280 mM, about 120 mM to about 250 mM, about 120 mM to about 220 mM, about 120 mM to about 200 mM, about 120 mM to about 180 mM, about 120 mM to about 150 mM, about 130 mM to about 400 mM, about 130 mM to about 300 mM, about 130 mM to about 280 mM, about 130 mM to about 250 mM, about 130 mM to about 220 mM, about 130 mM to about 200 mM, about 130 mM to about 180 mM, about 130 mM to about 150 mM, about 130 mM to about 140 mM, about 140 mM to about 400 mM, about 140 mM to about 300 mM, about 140 mM to about 280 mM, about 140 mM to about 250 mM, about 140 mM to about 220 mM, about 140 mM to about 200 mM, about 140 mM to about 180 mM, about 140 mM to about 150 mM, about 150 mM to about 400 mM, about 150 mM to about 300 mM, about 150 mM to about 250 mM, about 150 mM to about 200 mM, about 200 mM to about 400 mM, about 200 mM to about 350 mM, about 200 mM to about 300 mM, about 200 mM to about 250 mM, about 220 mM to about 400 mM, about 220 mM to about 350 mM, about 220 mM to about 300 mM, or about 220 mM to about 280 mM.

The concentration of the metal salt may be freely adjusted within a range that maintains the stability of the antibody, and may vary individually depending on the type of each specific metal salt. The concentration of the metal salt may be any range or any value selected about 0.1% (w/v) to about 20.0% (w/v). For example, the concentration of the metal salt may be about 0.1% (w/v) to about 20.0% (w/v), about 0.1% (w/v) to about 15.0% (w/v), about 0.1% (w/v) to about 12.0% (w/v), about 0.1% (w/v) to about 10.0% (w/v), about 0.1% (w/v) to about 8.0% (w/v), about 0.1% (w/v) to about 5.0% (w/v), about 0.1% (w/v) to about 3.0% (w/v), about 0.1% (w/v) to about 2.0% (w/v), about 0.1% (w/v) to about 1.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 1.0% (w/v) to about 8.0% (w/v), about 1.0% (w/v) to about 5.0% (w/v), about 1.0% (w/v) to about 3.0% (w/v), about 1.0% (w/v) to about 2.0% (w/v), about 2.0% (w/v) to about 20.0% (w/v), about 2.0% (w/v) to about 15.0% (w/v), about 2.0% (w/v) to about 12.0% (w/v), about 2.0% (w/v) to about 10.0% (w/v), about 2.0% (w/v) to about 8.0% (w/v), about 2.0% (w/v) to about 5.0% (w/v), about 3.0% (w/v) to about 20.0% (w/v), about 3.0% (w/v) to about 15.0% (w/v), about 3.0% (w/v) to about 12.0% (w/v), about 3.0% (w/v) to about 10.0% (w/v), about 3.0% (w/v) to about 9.0% (w/v), about 3.0% (w/v) to about 8.0% (w/v), about 3.0% (w/v) to about 7.0% (w/v), about 3.0% (w/v) to about 6.0% (w/v), about 3.0% (w/v) to about 5.0% (w/v), about 3.0% (w/v) to about 4.5% (w/v), about 3.0% (w/v) to about 4.0% (w/v), about 3.0% (w/v) to about 3.5% (w/v), about 4.0% (w/v) to about 20.0% (w/v), about 4.0% (w/v) to about 15.0% (w/v), about 4.0% (w/v) to about 12.0% (w/v), about 4.0% (w/v) to about 10.0% (w/v), about 4.0% (w/v) to about 9.0% (w/v), about 4.0% (w/v) to about 8.0% (w/v), about 4.0% (w/v) to about 7.0% (w/v), about 4.0% (w/v) to about 6.0% (w/v), about 4.0% (w/v) to about 5.0% (w/v), about 4.5% (w/v) to about 20.0% (w/v), about 4.5% (w/v) to about 15.0% (w/v), about 4.5% (w/v) to about 12.0% (w/v), about 4.5% (w/v) to about 10.0% (w/v), about 4.5% (w/v) to about 9.0% (w/v), about 4.5% (w/v) to about 8.0% (w/v), about 4.5% (w/v) to about 7.0% (w/v), about 4.5% (w/v) to about 6.0% (w/v), or about 4.5% (w/v) to about 5.0% (w/v).

In an embodiment, the stabilizer may comprise one or more selected from sucrose, trehalose, mannitol, arginine, lysine, histidine, methionine, glycine, and NaCl. In another embodiment, the stabilizer may comprise one or more selected from trehalose, mannitol, arginine, lysine, methionine, glycine, and NaCl.

In an embodiment, the stabilizer may comprise one or more selected from trehalose, mannitol, histidine, glycine, and methionine. In another embodiment, the stabilizer may comprise one or more selected from trehalose, mannitol, glycine, and methionine. In such embodiments, the liquid formulation may have an aggregation temperature (T_{agg}) of about 73.83 °C or higher.

In an embodiment, the stabilizer may comprise one or more selected from methionine, mannitol, and histidine. In another embodiment, the stabilizer may incldue one or more selected from glycine, methionine and mannitol. In such embodiments, the liquid formulation may have an aggregation temperature (T_{agg}) of about 74 °C or higher.

In certain embodiments, the stabilizer may comprise sucrose. The concentration of the sucrose may be about 0.1% (w/v) to about 20.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 5.0% (w/v) to about 20.0% (w/v), about 5.0% (w/v) to about 15.0% (w/v), about 5.0% (w/v) to about 12.0% (w/v), about 5.0% (w/v) to about 10.0% (w/v), about 8.0% (w/v) to about 20.0% (w/v), about 8.0% (w/v) to about 15.0% (w/v), about 8.0% (w/v) to about 14.0% (w/v), about 8.0% (w/v) to about 13.0% (w/v), about 8.0% (w/v) to about 12.0% (w/v), about 8.0% (w/v) to about 11.0% (w/v), about 8.0% (w/v) to about 10.0% (w/v), about 8.0% (w/v) to about 9.0% (w/v), or about 8.0% (w/v).

In certain embodiments, the stabilizer may comprise trehalose. The concentration of the trehalose may be about 0.1% (w/v) to about 20.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 5.0% (w/v) to about 20.0% (w/v), about 5.0% (w/v) to about 15.0% (w/v), about 5.0% (w/v) to about 12.0% (w/v), about 5.0% (w/v) to about 10.0% (w/v), about 8.0% (w/v) to about 20.0% (w/v), about 8.0% (w/v) to about 15.0% (w/v), about 8.0% (w/v) to about 14.0% (w/v), about 8.0% (w/v) to about 13.0% (w/v), about 8.0% (w/v) to about 12.0% (w/v), about 8.0% (w/v) to about 11.0% (w/v), about 8.0% (w/v) to about 10.0% (w/v), about 8.5% (w/v) to about 10.0% (w/v), about 8.0% (w/v) to about 9.5% (w/v), or about 9.0% (w/v).

In certain embodiments, the stabilizer may comprise mannitol. The concentration of the mannitol may be about 0.1% (w/v) to about 20.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 1.0% (w/v) to about 8.0% (w/v), about 1.0% (w/v) to about 5.0% (w/v), about 2.0% (w/v) to about 20.0% (w/v), about 2.0% (w/v) to about 15.0% (w/v), about 2.0% (w/v) to about 12.0% (w/v), about 2.0% (w/v) to about 10.0% (w/v), about 2.0% (w/v) to about 8.0% (w/v), about 2.0% (w/v) to about 5.0% (w/v), about 3.0% (w/v) to about 20.0% (w/v), about 3.0% (w/v) to about 15.0% (w/v), about 3.0% (w/v) to about 12.0% (w/v), about 3.0% (w/v) to about 10.0% (w/v), about 3.0% (w/v) to about 9.0% (w/v), about 3.0% (w/v) to about 8.0% (w/v), about 3.0% (w/v) to about 7.0% (w/v), about 3.0% (w/v) to about 6.0% (w/v), about 3.0% (w/v) to about 5.0% (w/v), about 3.0% (w/v) to about 4.5% (w/v), about 3.0% (w/v) to about 4.0% (w/v), about 3.0% (w/v) to about 3.5% (w/v), about 4.0% (w/v) to about 5.0% (w/v), about 4.2% (w/v) to about 4.7% (w/v), or about 3.13% (w/v), about 4.2% (w/v), or about 4.3% (w/v).

In certain embodiments, the stabilizer may comprise arginine. The concentration of the arginine may be about 1 mM to about 400 mM, about 10 mM to about 400 mM, about 20 mM to about 400 mM, about 50 mM to about 400 mM, about 50 mM to about 300 mM, about 50 mM to about 200 mM, about 50 mM to about 150 mM, about 80 mM to about 400 mM, about 80 mM to about 300 mM, about 80 mM to about 200 mM, about 80 mM to about 150 mM, about 100 mM to about 400 mM, about 100 mM to about 300 mM, about 100 mM to about 200 mM, about 100 mM to about 180 mM, about 100 mM to about 150 mM, about 120 mM to about 400 mM, about 120 mM to about 300 mM, about 120 mM to about 200 mM, about 120 mM to about 180 mM, about 120 mM to about 150 mM, about 130 mM to about 400 mM, about 130 mM to about 300 mM, about 130 mM to about 200 mM, about 130 mM to about 180 mM, about 130 mM to about 150 mM, or about 140 mM.

In certain embodiments, the stabilizer may comprise lysine. The concentration of the lysine may be about 1 mM to about 400 mM, about 10 mM to about 400 mM, about 20 mM to about 400 mM, about 50 mM to about 400 mM, about 50 mM to about 300 mM, about 50 mM to about 200 mM, about 50 mM to about 150 mM, about 80 mM to about 400 mM, about 80 mM to about 300 mM, about 80 mM to about 200 mM, about 80 mM to about 150 mM, about 100 mM to about 400 mM, about 100 mM to about 300 mM, about 100 mM to about 200 mM, about 100 mM to about 180 mM, about 100 mM to about 150 mM, about 120 mM to about 400 mM, about 120 mM to about 300 mM, about 120 mM to about 200 mM, about 120 mM to about 180 mM, about 120 mM to about 150 mM, about 130 mM to about 400 mM, about 130 mM to about 300 mM, about 130 mM to about 200 mM, about 130 mM to about 180 mM, about 130 mM to about 150 mM, about 130 mM to about 140 mM, or about 135 mM.

In certain embodiments, the stabilizer may comprise histidine. The concentration of the histidine may be about 1 mM to about 400 mM, about 10 mM to about 400 mM, about 20 mM to about 400 mM, about 30 mM to about 400 mM, about 30 mM to about 300 mM, about 30 mM to about 200 mM, about 30 mM to about 170 mM, about 30 mM to about 150 mM, about 30 mM to about 100 mM, about 30 mM to about 80 mM, about 30 mM to about 50 mM, about 30 mM to about 40 mM, about 40 mM to about 400 mM, about 40 mM to about 300 mM, about 40 mM to about 200 mM, about 40 mM to about 150 mM, about 40 mM to about 100 mM, about 40 mM to about 80 mM, about 40 mM to about 50 mM, about 50 mM to about 400 mM, about 50 mM to about 300 mM, about 50 mM to about 250 mM, about 50 mM to about 200 mM, about 50 mM to about 150 mM, about 50 mM to about 100 mM, about 50 mM to about 80 mM, about 60 mM to about 400 mM, about 60 mM to about 300 mM, about 60 mM to about 250 mM, about 60 mM to about 200 mM, about 60 mM to about 150 mM, about 60 mM to about 100 mM, about 60 mM to about 80 mM, or about 70 mM. Since histidine may also serve as a stabilizer in the formulation as its concentration increases, it may function as a dual-purpose agent acting as both a buffer and a stabilizer. The formulation according to an aspect may additionally comprise histidine as a stabilizer in addition to histidine as a buffer.

In certain embodiments, the stabilizer may comprise methionine. The concentration of the methionine may be about 1 mM to about 400 mM, about 1 mM to about 300 mM, about 1 mM to about 200 mM, about 1 mM to about 100 mM, about 1 mM to about 80 mM, about 1 mM to about 60 mM, about 1 mM to about 50 mM, about 1 mM to about 30 mM, about 10 mM to about 400 mM, about 20 mM to about 400 mM, about 20 mM to about 300 mM, about 20 mM to about 200 mM, about 20 mM to about 100 mM, about 20 mM to about 80 mM, about 20 mM to about 60 mM, about 20 mM to about 50 mM, about 20 mM to about 30 mM, about 30 mM to about 400 mM, about 30 mM to about 300 mM, about 30 mM to about 200 mM, about 30 mM to about 150 mM, about 30 mM to about 100 mM, about 30 mM to about 80 mM, about 30 mM to about 60 mM, about 30 mM to about 50 mM, about 50 mM to about 400 mM, about 50 mM to about 300 mM, about 50 mM to about 250 mM, about 50 mM to about 200 mM, about 50 mM to about 150 mM, about 50 mM to about 100 mM, about 60 mM to about 400 mM, about 60 mM to about 300 mM, about 60 mM to about 250 mM, about 60 mM to about 200 mM, about 60 mM to about 150 mM, about 60 mM to about 100 mM, about 70 mM to about 400 mM, about 70 mM to about 300 mM, about 70 mM to about 250 mM, about 70 mM to about 200 mM, about 70 mM to about 150 mM, about 70 mM to about 100 mM, about 80 mM to about 400 mM, about 80 mM to about 300 mM, about 80 mM to about 250 mM, about 80 mM to about 200 mM, about 80 mM to about 150 mM, about 80 mM to about 120 mM, about 80 mM to about 100 mM, or about 90 mM.

In certain embodiments, the stabilizer may comprise glycine. The concentration of the glycine may be about 1 mM to about 400 mM, about 10 mM to about 400 mM, about 50 mM to about 400 mM, about 100 mM to about 400 mM, about 100 mM to about 300 mM, about 100 mM to about 280 mM, about 100 mM to about 250 mM, about 150 mM to about 400 mM, about 150 mM to about 300 mM, about 150 mM to about 250 mM, about 200 mM to about 400 mM, about 200 mM to about 350 mM, about 200 mM to about 300 mM, about 200 mM to about 250 mM, about 220 mM to about 400 mM, about 220 mM to about 350 mM, about 220 mM to about 300 mM, about 220 mM to about 280 mM, about 250 mM to about 400 mM, about 250 mM to about 300 mM, or about 250 mM or about 280 mM.

In certain embodiments, the stabilizer may comprise NaCl. The concentration of the NaCl may be about 0.1% (w/v) to about 20.0% (w/v), about 0.1% (w/v) to about 15.0% (w/v), about 0.1% (w/v) to about 12.0% (w/v), about 0.1% (w/v) to about 10.0% (w/v), about 0.1% (w/v) to about 8.0% (w/v), about 0.1% (w/v) to about 5.0% (w/v), about 0.1% (w/v) to about 3.0% (w/v), about 0.1% (w/v) to about 2.0% (w/v), about 0.1% (w/v) to about 1.0% (w/v), about 1.0% (w/v) to about 20.0% (w/v), about 1.0% (w/v) to about 15.0% (w/v), about 1.0% (w/v) to about 12.0% (w/v), about 1.0% (w/v) to about 10.0% (w/v), about 1.0% (w/v) to about 8.0% (w/v), about 1.0% (w/v) to about 5.0% (w/v), about 1.0% (w/v) to about 3.0% (w/v), about 1.0% (w/v) to about 2.0% (w/v), about 2.0% (w/v) to about 20.0% (w/v), about 2.0% (w/v) to about 15.0% (w/v), about 2.0% (w/v) to about 12.0% (w/v), about 2.0% (w/v) to about 10.0% (w/v), about 2.0% (w/v) to about 8.0% (w/v), about 2.0% (w/v) to about 5.0% (w/v), about 3.0% (w/v) to about 20.0% (w/v), about 3.0% (w/v) to about 15.0% (w/v), about 3.0% (w/v) to about 12.0% (w/v), about 3.0% (w/v) to about 10.0% (w/v), about 3.0% (w/v) to about 9.0% (w/v), about 3.0% (w/v) to about 8.0% (w/v), about 3.0% (w/v) to about 7.0% (w/v), about 3.0% (w/v) to about 6.0% (w/v), about 3.0% (w/v) to about 5.0% (w/v), about 3.0% (w/v) to about 4.5% (w/v), about 3.0% (w/v) to about 4.0% (w/v), about 3.0% (w/v) to about 3.5% (w/v), about 4.0% (w/v) to about 20.0% (w/v), about 4.0% (w/v) to about 15.0% (w/v), about 4.0% (w/v) to about 12.0% (w/v), about 4.0% (w/v) to about 10.0% (w/v), about 4.0% (w/v) to about 9.0% (w/v), about 4.0% (w/v) to about 8.0% (w/v), about 4.0% (w/v) to about 7.0% (w/v), about 4.0% (w/v) to about 6.0% (w/v), about 4.0% (w/v) to about 5.0% (w/v), about 4.5% (w/v) to about 20.0% (w/v), about 4.5% (w/v) to about 15.0% (w/v), about 4.5% (w/v) to about 12.0% (w/v), about 4.5% (w/v) to about 10.0% (w/v), about 4.5% (w/v) to about 9.0% (w/v), about 4.5% (w/v) to about 8.0% (w/v), about 4.5% (w/v) to about 7.0% (w/v), about 4.5% (w/v) to about 6.0% (w/v), or about 4.5% (w/v) to about 5.0% (w/v).

In an embodiment, the liquid formulation may comprise at least one amino acid. Specifically, the liquid formulation may comprise at least one amino acid as a buffer or as a stabilizer, and the total number of moles of amino acids comprised in the formulation may be 20 moles or more based on 1 mole of antibody. In other words, the liquid formulation may comprise at least one amino acid in an amount of 20 moles or more, for example, 25 moles or more, 27 moles or more, 29 moles or more, 30 moles or more, 40 moles or more, 50 moles or more, 60 moles or more, 70 moles or more, 20 moles to 700 moles, 20 moles to 600 moles, 20 moles to 500 moles, 20 moles to 450 moles, 20 moles to 440 moles, 25 moles to 700 moles, 25 moles to 600 moles, 25 moles to 500 moles, 25 moles to 450 moles, 25 moles to 440 moles, 29 moles to 700 moles, 29 moles to 600 moles, 29 moles to 500 moles, 29 moles to 450 moles, 29 moles to 440 moles, 40 moles to 700 moles, 40 moles to 600 moles, 40 moles to 500 moles, 40 moles to 450 moles, 40 moles to 440 moles, 70 moles to 700 moles, 70 moles to 600 moles, 70 moles to 500 moles, 70 moles to 450 moles, or 70 moles to 440 moles, relative to 1 mole of the antibody.

### (4) Surfactant

A liquid formulation according to an aspect may optionally comprise a surfactant.

Accordingly, in an embodiment, the liquid formulation may be a liquid formulation comprising: an anti-IL-23 antibody; a buffer; a stabilizer comprising at least one selected from sugars, sugar alcohols, amino acids, metal salts, salts thereof, and hydrates thereof; and a surfactant, wherein the pH is about 4.0 to about 8.5. In another embodiment, the liquid formulation may be a liquid formulation comprising: an anti-IL-23 antibody; a buffer; and a stabilizer comprising at least one selected from sugars, sugar alcohols, amino acids, metal salts, salts thereof, and hydrates thereof, and not comprising a surfactant, wherein the pH is about 4.0 to about 8.5.

The surfactant may be selected from any pharmaceutically acceptable surfactants capable of evenly dispersing a protein (e.g., antibody) in a liquid formulation medium.

The surfactant may be a non-ionic surfactant.

Specifically, the surfactant may be at least one selected from the group consisting of polysorbates, poloxamers, sorbitan esters of other fatty acids, polyethylene-polypropylene glycols, polyoxyethylene compounds, sodium dodecyl sulfate (SDS), and the like.

The polysorbate may comprise polysorbate 20, polysorbate 40, polysorbate 60, polysorbate 65, polysorbate 80, polysorbate 85, and the like.

The poloxamer may comprise a PEO-PPO-PEO copolymer (where PEO is poly(ethylene oxide) and PPO is poly(propylene oxide)), and the like.

The sorbitan esters of other fatty acids may refer to sorbitan esters of other fatty acids other than those in polysorbates, and may comprise, for example, sorbitan polyethoxylates and the like.

The polyoxyethylene compound may comprise polyoxyethylene-stearate, polyoxyethylene alkyl ether (alkyl: C1-C30), polyoxyethylene monolyl ether, alkylphenyl polyoxyethylene copolymer (alkyl: C1-C30), and the like.

In an embodiment, the surfactant may comprise one or more selected from polysorbate 20, polysorbate 40, polysorbate 60, polysorbate 65, polysorbate 80, and polysorbate 85.

In an embodiment, the surfactant may comprise polysorbate 20, polysorbate 80, or a combination thereof.

In certain embodiments, the surfactant may comprise polysorbate 80.

The concentration of the surfactant may be any range or any value selected about 0.01% (w/v) to about 0.9% (w/v). For example, the concentration of the surfactant may be about 0.01% (w/v) to about 0.9% (w/v), about 0.01% (w/v) to about 0.5% (w/v), about 0.01% (w/v) to about 0.1% (w/v), about 0.01% (w/v) to about 0.08% (w/v), about 0.01% (w/v) to about 0.06% (w/v), about 0.03% (w/v) to about 0.9% (w/v), about 0.03% (w/v) to about 0.5% (w/v), about 0.03% (w/v) to about 0.1% (w/v), about 0.03% (w/v) to about 0.08% (w/v), about 0.03% (w/v) to about 0.07% (w/v), about 0.03% (w/v) to about 0.06% (w/v), about 0.05% (w/v) to about 0.9% (w/v), about 0.05% (w/v) to about 0.5% (w/v), about 0.05% (w/v) to about 0.1% (w/v), about 0.05% (w/v) to about 0.08% (w/v), about 0.05% (w/v) to about 0.07% (w/v), or about 0.05% (w/v) to about 0.06% (w/v).

In certain embodiments, the concentration of the surfactant may be about 0.05% (w/v).

### (5) Diluent

A liquid formulation according to an aspect may additionally comprise a diluent.

The diluent may be an aqueous carrier. The aqueous carrier may be a pharmaceutically acceptable carrier that is safe and non-toxic when administered to humans, and may be, for example, water, saline solution, Ringer's solution, dextrose, or a mixture thereof.

In an embodiment, the diluent may be water. The water may be water in a standard state. Accordingly, the liquid formulation may be an aqueous liquid formulation.

The term "standard state" refers to a reference that specifies the temperature and pressure used to describe a solution having a specific composition, and may mean, for example, a temperature of 25°C±2°C and a pressure of 1 atmosphere. Those skilled in the art will recognize that liquid formulations equivalent to those disclosed herein may be produced at other temperatures and pressures. Whether such a liquid formulation is equivalent to that disclosed herein may be determined under standard state conditions.

### (6) Stability

A liquid formulation according to an aspect may be a biosimilar to Tremfya^{®}. Accordingly, the liquid formulation according to an aspect has improved stability compared to the Tremfya^{®} formulation.

The term "biosimilar," also called "biogeneric," refers to a generic version of an original biopharmaceutical. Since biopharmaceuticals are produced through cells rather than synthetic chemical products, it is impossible to replicate a product that is perfectly identical to the original medicament. Therefore, a generic version of a biopharmaceutical is called a biosimilar in the sense that it is similar, though not identical, to the original medicament.

The term "stability" means that an antibody (e.g., guselkumab) contained in the formulation substantially retains its physical stability, chemical stability, and/or biological activity before and after administration, during additional manufacturing processes, or during storage or preservation. Physical stability, chemical stability and/or biological activity may be evaluated by conventionally known methods. Methods for evaluating the stability of an antibody contained in a formulation in a short period of time include measuring the aggregation temperature (T_{agg}), diffusion interaction parameter (K_{D}), and the like, after exposing the formulation to heat (temperature) or chemicals, which are factors that cause structural and physicochemical denaturation of proteins. For example, "improved stability" may mean that the T_{agg} or K_{D} value measured for the formulation is increased by 0.01% or more, 0.1% or more, 0.5% or more, 1% or more, 2% or more, 3% or more, 4% or more, 5% or more, 10% or more, 15% or more, or 20% or more, compared to an existing formulation. An increase in T_{agg} or K_{D} value may mean that stability is improved as the degree of aggregation or denaturation of the antibody in the formulation due to external factors (e.g., heat, temperature, chemical substances) is decreased.

The liquid formulation may inhibit aggregation of the antibody.

The liquid formulation may have an increased aggregation temperature (T_{agg}).

The liquid formulation may have an increased T_{agg} compared to a formulation having the same buffer, pH and stabilizer conditions as Tremfya^{®}. The liquid formulation may have an increased T_{agg} compared to a liquid formulation including 11 mM histidine as a buffer, having a pH of 5.8, and including 7.9% (w/v) sucrose as a stabilizer. The liquid formulation may have an increased T_{agg} compared to Tremfya^{®}.

The liquid formulation may have an aggregation temperature (T_{agg}) of higher than 73.82 °C, about 73.83 °C or higher, about 73.9 °C or higher, or about 74 °C or higher.

In an embodiment, the liquid formulation has an aggregation temperature (T_{agg}) of about 73.83 °C or higher, and may be selected from the following:
A) the buffer comprises acetate, and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

In an embodiment, the liquid formulation has an aggregation temperature (T_{agg}) of about 73.9 °C or higher, and may be selected from the following:
A) the buffer comprises acetate, and the pH is about 4.0 to about 6.0; or
B) the buffer comprises histidine, and the pH is about 5.7 to about 7.0.

In an embodiment, the liquid formulation has an aggregation temperature (T_{agg}) of about 73.83 °C or higher, and may be selected from the following:
A) the stabilizer comprises trehalose;
B) the stabilizer comprises mannitol;
C) the stabilizer comprises arginine;
D) the stabilizer comprises lysine;
E) the stabilizer comprises histidine;
F) the stabilizer comprises methionine;
G) the stabilizer comprises glycine; or
H) the stabilizer comprises NaCl.

In an embodiment, the liquid formulation has an aggregation temperature (T_{agg}) of about 74 °C or higher, and may be selected from the following:
A) the stabilizer comprises trehalose;
B) the stabilizer comprises mannitol;
C) the stabilizer comprises methionine; or
D) the stabilizer comprises glycine.

The liquid formulation may have an increased diffusion interaction parameter (K_{D}). The liquid formulation may have an increased K_{D} compared to a formulation having the same buffer, pH and stabilizer conditions as Tremfya^{®}. The liquid formulation may have an increased K_{D} compared to a liquid formulation including 11 mM histidine as a buffer, having a pH of 5.8, and comprising 7.9% (w/v) sucrose as a stabilizer.

The liquid formulation may have an increased K_{D} compared to Tremfya^{®}.

The liquid formulation may have a diffusion interaction parameter (K_{D}) of higher than about 19.9 mL/g, about 20.0 mL/g or higher, about 20.7 mL/g or higher, about 20.8 mL/g or higher, about 20.9 mL/g or higher, about 21.0 mL/g or higher, about 21.1 mL/g or higher, or about 30.0 mL/g or higher.

In an embodiment, the liquid formulation has a diffusion interaction parameter (K_{D}) of about 20.0 mL/g or higher, and may be selected from the following:
A) the buffer comprises acetate, and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

In an embodiment, the liquid formulation has a diffusion interaction parameter (K_{D}) of about 21.0 mL/g or higher, and may be selected from the following:
A) the buffer comprises acetate, and the pH is about 4.0 to about 5.3; or
B) the buffer comprises histidine, and the pH is about 5.7 to about 7.0.

### (7) Formulation

The term "liquid formulation" refers to a formulation in a liquid state.

A liquid formulation according to an aspect is a stable liquid formulation of an anti-IL-23 antibody. The liquid formulation may have improved stability compared to a Tremfya^{®} formulation.

A liquid formulation according to an aspect may be selected from the following items:
1) A liquid formulation comprising an anti-IL-23 antibody (e.g., guselkumab) and a buffer, wherein the pH is about 4.0 to about 8.5;
2) A liquid formulation comprising an anti-IL-23 antibody (e.g., guselkumab) and at least one selected from acetate, a salt thereof, and a hydrate thereof as a buffer, wherein the pH is 4.0 to 6.0;
3) A liquid formulation comprising an anti-IL-23 antibody (e.g., guselkumab) and at least one selected from histidine, a salt thereof, and a hydrate thereof as a buffer, wherein the pH is 5.0 to 7.0;
4) A liquid formulation comprising an anti-IL-23 antibody (e.g., guselkumab) and at least one selected from phosphate, a salt thereof, and a hydrate thereof as a buffer, wherein the pH is 6.0 to 8.0;
5) A liquid formulation comprising an anti-IL-23 antibody (e.g., guselkumab) and at least one selected from citrate, its salts and its hydrates as a buffer, wherein the pH is 4.5 to 6.5;
6) A liquid formulation comprising an anti-IL-23 antibody (e.g., guselkumab) and at least one selected from succinate, a salt thereof, and a hydrate thereof as a buffer, wherein the pH is 4.5 to 6.0;
7) A liquid formulation comprising an anti-IL-23 antibody (e.g., guselkumab) and at least one selected from acetate, a salt thereof, and a hydrate thereof as a buffer, wherein the pH is 4.0 to 6.5, 4.0 to 5.6, 4.0 to 5.2, 5.8, or 6.0;
8) A liquid formulation comprising an anti-IL-23 antibody (e.g., guselkumab) and at least one selected from histidine, a salt thereof, and a hydrate thereof as a buffer, wherein the pH is 4.8 to 7.2, 5.6 to 7.2, 5.7 to 7.0, 5.7 to 6.7, 5.8, 6.0, 6.5, or 7.0;
9) A liquid formulation comprising an anti-IL-23 antibody (e.g., guselkumab) and at least one selected from phosphate, a salt thereof, and a hydrate thereof as a buffer, wherein the pH is 5.8 to 8.2, or 5.8 to 6.7;
10) A liquid formulation comprising an anti-IL-23 antibody (e.g., guselkumab) and at least one selected from citrate, its salts and its hydrates as a buffer, wherein the pH is 4.5 to 7.0, or 5.5 to 6.5;
11) A liquid formulation comprising an anti-IL-23 antibody (e.g., guselkumab) and at least one selected from succinate, a salt thereof, and a hydrate thereof as a buffer, wherein the pH is 4.3 to 6.5;
12) A liquid formulation comprising an anti-IL-23 antibody (e.g., guselkumab) and at least one selected from acetate, histidine, salts thereof and hydrates thereof as a buffer, wherein the pH is 4.0 to 8.5;
13) A liquid formulation comprising an anti-IL-23 antibody (e.g., guselkumab), a buffer, and a stabilizer, wherein the pH is 4.0 to 8.5;
14) A liquid formulation comprising an anti-IL-23 antibody (e.g., guselkumab), a buffer, and sucrose as a stabilizer, wherein the pH is 4.0 to 8.5;
15) A liquid formulation comprising an anti-IL-23 antibody (e.g., guselkumab), a buffer, and trehalose as a stabilizer, wherein the pH is 4.0 to 8.5;
16) A liquid formulation comprising an anti-IL-23 antibody (e.g., guselkumab), a buffer, and mannitol as a stabilizer, wherein the pH is 4.0 to 8.5;
17) A liquid formulation comprising an anti-IL-23 antibody (e.g., guselkumab), a buffer, and arginine as a stabilizer, wherein the pH is 4.0 to 8.5;
18) A liquid formulation comprising an anti-IL-23 antibody (e.g., guselkumab), a buffer, and lysine as a stabilizer, wherein the pH is 4.0 to 8.5;
19) A liquid formulation comprising an anti-IL-23 antibody (e.g., guselkumab), a buffer, and histidine as a stabilizer, wherein the pH is 4.0 to 8.5;
20) A liquid formulation comprising an anti-IL-23 antibody (e.g., guselkumab), a buffer, and methionine as a stabilizer, wherein the pH is 4.0 to 8.5;
21) A liquid formulation comprising an anti-IL-23 antibody (e.g., guselkumab), a buffer, and glycine as a stabilizer, wherein the pH is 4.0 to 8.5;
22) A liquid formulation comprising an anti-IL-23 antibody (e.g., guselkumab), a buffer, and NaCl as a stabilizer, wherein the pH is 4.0 to 8.5;
23) A liquid formulation comprising an anti-IL-23 antibody (e.g., guselkumab), a buffer, and at least one selected from trehalose, mannitol, histidine, methionine and glycine as a stabilizer, wherein the pH is 4.0 to 8.5;
24) The liquid formulation of one of items 13 to 23, wherein the buffer and the pH have the combination of the buffer and the pH of any one of items 1 to 12;
25) The liquid formulation of any one of items 1 to 24, optionally comprising a surfactant;
26) The liquid formulation of item 25, wherein the surfactant comprises at least one selected from polysorbates, poloxamers, and sorbitan esters of other fatty acids;
27) The liquid formulation of item 25, wherein the surfactant comprises polysorbate 20, polysorbate 80, or a combination thereof;
28) The liquid formulation of item 25, wherein the surfactant comprises polysorbate 80;
29) The liquid formulation of any one of items 25 to 28, wherein a concentration of the surfactant is from 0.01% (w/v) to 0.9% (w/v);
30) The liquid formulation of any one of items 1 to 29, further comprising a diluent;
31) The liquid formulation of item 30, wherein the diluent is water;
32) The liquid formulation of any one of items 1 to 31, which is an aqueous liquid formulation; or
33) The liquid formulation of any one of items 1 to 32, which is for subcutaneous injection.

### (8) Device

Another aspect provides a device comprising a liquid formulation of the aspect.

The device is primarily used for parenteral administration (e.g., subcutaneous, intramuscular, intravenous, intraperitoneal, intracerebrospinal, intraarticular, intrasynovial, and/or intrathecal administration). The device may be accompanied by instructions for administration.

The device may comprise the liquid formulation in a container selected from a syringe, a pre-filled syringe, an auto-injector, a bottle, a vial, and a tube.

In an embodiment, the liquid formulation may be comprised in a pre-filled syringe. The pre-filled syringe may be a single-dose pre-filled syringe.

### (9) Treatment of Disease

Another aspect provides a method of treating an IL-23 related condition, comprising administering the liquid formulation of the aspect to a subject in need thereof. The liquid formulation may be in a form comprised in a device.

Another aspect provides a use of a liquid formulation of the aspect in the manufacture of a medicament for treating an IL-23 related condition.

The method of treating the IL-23 related condition may further comprise, prior to the administering, identifying a subject in need of administration of an anti-IL-23 antibody (e.g., guselkumab).

The subject may be a subject in need of administration of a liquid formulation containing the anti-IL-23 antibody (e.g., guselkumab). A subject in need of administration of a liquid formulation containing the anti-IL-23 antibody (e.g., guselkumab) may be a subject having a disease or disorder that may be significantly treated (e.g., elimination, reduction, alleviation, or improvement of symptoms, etc.) by administration of an anti-IL-23 antibody. The subject may be selected from mammals including humans.

The liquid formulation may be administered in a pharmaceutically effective amount.

The IL-23 related condition may comprise any condition or disease that may be treated by administration of an anti-IL-23 antibody. The IL-23 related condition treatable by administration of an anti-IL-23 antibody (e.g., guselkumab) may comprise all indications previously approved or to be approved in the future for the anti-IL-23 antibody (e.g., guselkumab).

The IL-23 related condition may be any one selected from psoriasis, psoriatic arthritis, palmoplantar pustulosis, Crohn's disease (CD), inflammatory bowel disease (IBD), multiple sclerosis (MS), ulcerative colitis (UC), and ankylosing spondylitis.

The psoriasis may be plaque psoriasis. The plaque psoriasis may be moderate to severe plaque psoriasis.

The psoriatic arthritis may be active psoriatic arthritis (PsA).

The ulcerative colitis may be moderately to severely active ulcerative colitis.

### (10) Route of Administration and Dosage

A liquid formulation according to an aspect may be administered by a parenteral route. The parenteral route may comprise subcutaneous administration, intravenous administration, and the like. Parenteral administration may be by bolus injection or continuous infusion.

In certain embodiments, the liquid formulation may be for subcutaneous injection.

The liquid formulation may be formulated into a formulation suitable for the administration route. For example, the liquid formulation may be formulated as an injection, an injectable ready-to-use form, or the like, but is not limited thereto.

The liquid formulation may be formulated such that the total amount or a pharmaceutically effective amount of the anti-IL-23 antibody (e.g., guselkumab) is contained in a single formulation, or divided into two or more formulations (e.g., 2, 3, 4, 5, 6, 7, 8, 9, or 10 formulations). The liquid formulation may be contained in a single-dose form of a device.

The liquid formulation may be administered such that the total amount of the anti-IL-23 antibody (e.g., guselkumab) contained in a single formulation is administered into the body at once (e.g., within 1 minute, within 30 seconds, within 20 seconds, or within 10 seconds); or is administered slowly into the body over a period of 5 minutes or more, 10 minutes or more, 30 minutes or more, 60 minutes or more, 90 minutes or more, 120 minutes or more, 150 minutes or more, 180 minutes or more, 210 minutes or more, or 240 minutes or more, but is not limited thereto.

The subject to be administered the liquid formulation may be selected from mammals including primates (e.g., humans, etc.), rodents (e.g., mice, rats, guinea pigs, hamsters, rabbits, etc.), cats, dogs, pigs, cows, horses, and the like.

A pharmaceutically effective amount of the liquid formulation or the anti-IL-23 antibody (e.g., guselkumab) contained therein may refer to a content or dosage capable of exhibiting a desired pharmacological effect, for example, the elimination, reduction, alleviation, or improvement of symptoms. The pharmaceutically effective amount may be determined in various ways depending on factors such as the formulation method, administration mode, age, weight, sex, pathological condition (severity of the condition) of the patient, food, administration time, administration interval, administration route, excretion rate, response sensitivity, previous therapy, and clinical history. The dosage may be adjusted according to the judgment of a physician in charge. The pharmaceutically effective amount may be administered at once or may be administered in multiple divided doses of two or more.

For example, the liquid formulation may be administered at a dose such that the amount of the anti-IL-23 antibody (e.g., guselkumab) is about 1 mg to about 500 mg, about 1 mg to about 250 mg, about 1 mg to about 200 mg, about 1 mg to about 175 mg, about 1 mg to about 150 mg, about 1 mg to about 125 mg, about 1 mg to about 100 mg, about 1 mg to about 75 mg, about 1 mg to about 50 mg, about 1 mg to about 30 mg, about 1 mg to about 20 mg, about 1 mg to about 10 mg, about 2 mg to about 500 mg, about 2 mg to about 400 mg, about 2 mg to about 300 mg, about 2 mg to about 250 mg, about 2 mg to about 200 mg, about 2 mg to about 175 mg, about 2 mg to about 150 mg, about 2 mg to about 125 mg, about 2 mg to about 100 mg, about 2 mg to about 75 mg, about 2 mg to about 50 mg, about 2 mg to about 30 mg, about 2 mg to about 20 mg, about 2 mg to about 10 mg, about 5 mg to about 500 mg, about 5 mg to about 400 mg, about 5 mg to about 300 mg, about 5 mg to about 250 mg, about 5 mg to about 200 mg, about 5 mg to about 175 mg, about 5 mg to about 150 mg, about 5 mg to about 125 mg, about 5 mg to about 100 mg, about 5 mg to about 75 mg, about 5 mg to about 50 mg, about 5 mg to about 30 mg, about 5 mg to about 20 mg, about 5 mg to about 10 mg, about 10 mg to about 500 mg, about 10 mg to about 400 mg, about 10 mg to about 300 mg, about 10 mg to about 250 mg, about 10 mg to about 200 mg, about 10 mg to about 175 mg, about 10 mg to about 150 mg, about 10 mg to about 125 mg, about 10 mg to about 100 mg, about 10 mg to about 75 mg, about 10 mg to about 50 mg, about 10 mg to about 30 mg, about 10 mg to about 20 mg, about 25 mg to about 500 mg, about 25 mg to about 400 mg, about 25 mg to about 300 mg, about 25 mg to about 250 mg, about 25 mg to about 200 mg, about 25 mg to about 175 mg, about 25 mg to about 150 mg, about 25 mg to about 125 mg, about 25 mg to about 100 mg, about 25 mg to about 75 mg, about 25 mg to about 50 mg, about 25 mg to about 30 mg, about 50 mg to about 500 mg, about 50 mg to about 400 mg, about 50 mg to about 300 mg, about 50 mg to about 250 mg, about 50 mg to about 200 mg, about 50 mg to about 175 mg, about 50 mg to about 150 mg, about 50 mg to about 125 mg, about 50 mg to about 100 mg, about 50 mg to about 75 mg, about 75 mg to about 500 mg, about 75 mg to about 400 mg, about 75 mg to about 300 mg, about 75 mg to about 250 mg, about 75 mg to about 200 mg, about 75 mg to about 175 mg, about 75 mg to about 150 mg, about 75 mg to about 125 mg, about 75 mg to about 100 mg, about 90 mg to about 500 mg, about 90 mg to about 400 mg, about 90 mg to about 300 mg, about 90 mg to about 250 mg, about 90 mg to about 200 mg, about 90 mg to about 175 mg, about 90 mg to about 150 mg, about 90 mg to about 125 mg, about 90 mg to about 110 mg, about 90 mg to about 100 mg, about 100 mg to about 500 mg, about 100 mg to about 400 mg, about 100 mg to about 300 mg, about 100 mg to about 250 mg, about 100 mg to about 200 mg, about 100 mg to about 175 mg, about 100 mg to about 150 mg, about 100 mg to about 125 mg, about 100 mg to about 110 mg, about 500 mg, about 400 mg, about 300 mg, about 200 mg, about 150 mg, about 100 mg, about 50 mg, about 25 mg, about 10 mg, about 8 mg, about 6 mg, about 5 mg, about 4 mg, or about 2 mg, once every 4 to 8 weeks over a period of 4 weeks or longer.

The liquid formulation may be prepared as a general bulk formulation, and the components of the liquid formulation may be adjusted to a concentration higher than the concentration required for administration and then suitably diluted before use.

### Advantageous Effects

A liquid formulation of an anti-IL-23 antibody according to an aspect may have improved stability compared to existing commercially available formulations. Accordingly, the liquid formulation may be effectively used as a medicament for treating IL-23 related conditions.

### Description of Drawings

FIG. 1 is a graph showing the results of measuring T_{agg} for 24 formulations in which different buffer types and pHs are combined in Preparation Example 1, and for one formulation having the same buffer and pH conditions as Tremfya^{®}.
FIG. 2 is a graph showing the results of measuring K_{D} for 24 formulations in which different buffer types and pHs are combined in Preparation Example 1, and for one formulation having the same buffer and pH conditions as Tremfya^{®}.
FIG. 3 is a graph showing the results of measuring %HMW (initial) through SEC analysis for 24 formulations in which different buffer types and pHs are combined in Preparation Example 1, and for one formulation having the same buffer and pH conditions as Tremfya^{®}.
FIG. 4 is a graph showing the results of measuring T_{agg} for 10 formulations having various types of stabilizers in Preparation Example 2, and for one formulation having the same stabilizer conditions as Tremfya^{®}.

### Mode for Invention

Hereinafter, the present disclosure will be described in more detail with reference to the following examples. However, the following examples are only intended to illustrate the present disclosure and the scope of the present disclosure is not limited thereto.

### [Experimental Method]

### 1. Dynamic Light Scattering (DLS) Measurement for T_{agg}

Aggregates in samples were inspected by dynamic light scattering measurement. Specifically, each sample was diluted using its respective buffer and loaded into a 96-well plate. The plate was loaded into a DynoPro^{®} Plate ReaderTM II (Wyatt Technology) instrument. The size of aggregates within the formulation was measured via DLS while increasing the temperature from 25 to 80 °C at a rate of 0.25 °C/min. Stability was evaluated by measuring the temperature at which the size of the aggregates began to change, and a higher aggregation temperature (T_{agg}) value indicates a more stable the formulation.

### 2. Dynamic Light Scattering (DLS) Measurement for K_{D} (Diffusion Interaction Parameter)

The diffusion interaction parameter (K_{D}) was analyzed to evaluate whether the binding strength in the fluid phase is attractive or repulsive as the protein concentration increases when a sample is exposed to room temperature. This parameter evaluates the binding strength between substances that leads to irreversible aggregation, enabling the evaluation or ranking of superior formulations based on their aggregation tendencies. Specifically, each sample was diluted using a buffer (2, 4, 6, 8, 10 mg/mL) and loaded into a 96-well plate. The plate was loaded into a DynaPro^{®} Plate ReaderTM II (Wayatt Technology) instrument. After measuring the diffusion coefficient value according to the protein concentration of the prepared sample, K_{D} was calculated using the slope and y-intercept of the corresponding graph. A positive value indicates that repulsion between substances is dominant, resulting in a weak aggregation tendency, whereas a negative value indicates that attraction is dominant, resulting in a strong aggregation tendency. Thus, a larger positive K_{D} value indicates a more stable formulation.

### 3. Size Exclusion Chromatography (SEC)

The purity of the samples was inspected by size exclusion chromatography. In size exclusion chromatography, the percentages of antibody monomers, high molecular weight species (HMW), and low molecular weight species (LMW) are determined. In size exclusion chromatography, LMW elutes later than HMW. The presence of HMW indicates protein aggregation, and the presence of LMW indicates protein fragments.

### 4. Viscosity Measurement

The viscosity of each sample was measured at 20 °C using an Initium (High-Throughput Viscometer) instrument from Rheosense. Viscosity was measured using a chip (B-chip) equipped with a sensor capable of measuring a range of 0.2 to 3000 cP. For the same sample, measurements were taken over 17 segments, and the viscosity value of each sample was calculated by obtaining the average of the values where the slope fit R² value was 0.9995 or higher.

### [Preparation Examples]

Tremfya^{®} is a liquid formulation including 100 mg/ml guselkumab, 11 mM histidine, 7.9% (w/v) sucrose, 0.05% (w/v) polysorbate 80 (PS80), at pH 5.8.

Various formulations were prepared by changing the combination of buffer types and pH, or by changing the type of stabilizers in the Tremfya^{®} formulation**.**

### Preparation Example 1. Preparation of Formulations with Various Buffer Types and pH Combinations

Using guselkumab (CAS No. 1350289-85-8) as an anti-IL-23 antibody, aqueous liquid formulations having the composition shown in Table 1 below were prepared.

Table 1 shows the compositions of 24 formulations (No. 1-1 to 1-24) prepared by combining the types and pH of buffers used in approved biopharmaceuticals, and one formulation (No. 1-25) having the same buffer, pH, and stabilizer conditions as Tremfya^{®}.

The concentration of the antibody was included in a range of 2 to 10 mg/mL and was set differently according to the concentration required for each analysis method. Specifically, the antibody concentration of each formulation was 5 mg/ml for T_{agg} analysis; 2, 4, 6, 8, or 10 mg/ml for K_{D} analysis; and 10 mg/ml for %HMW analysis.

**[Table 1]**

| No. | Antibody concentration (mg/ml) | Buffer | pH | Stabilizer | Surfactant |
|---|---|---|---|---|---|
| 1-1 | 2 to 10 | 20 mM acetate | 4.0 | - | - |
| 1-2 | | 20 mM acetate | 4.5 | | |
| 1-3 | | 20 mM acetate | 5.0 | | |
| 1-4 | | 20 mM acetate | 5.5 | | |
| 1-5 | | 20 mM acetate | 6.0 | | |
| 1-6 | | 20 mM histidine | 5.0 | | |
| 1-7 | | 20 mM histidine | 5.5 | | |
| 1-8 | | 20 mM histidine | 6.0 | | |
| 1-9 | | 20 mM histidine | 6.5 | | |
| 1-10 | | 20 mM histidine | 7.0 | | |
| 1-11 | | 20 mM phosphate | 6.0 | | |
| 1-12 | | 20 mM phosphate | 6.5 | | |
| 1-13 | | 20 mM phosphate | 7.0 | | |
| 1-14 | | 20 mM phosphate | 7.5 | | |
| 1-15 | | 20 mM phosphate | 8.0 | | |
| 1-16 | | 20 mM citrate | 4.5 | | |
| 1-17 | | 20 mM citrate | 5.0 | | |
| 1-18 | | 20 mM citrate | 5.5 | | |
| 1-19 | | 20 mM citrate | 6.0 | | |
| 1-20 | | 20 mM citrate | 6.5 | | |
| 1-21 | | 20 mM succinate | 4.5 | | |
| 1-22 | | 20 mM succinate | 5.0 | | |
| 1-23 | | 20 mM succinate | 5.5 | | |
| 1-24 | | 20 mM succinate | 6.0 | | |
| 1-25 | 2 to 10 | 11 mM histidine | 5.8 | 7.9% (w/v) sucrose | - |

| | | | | | |
|---|---|---|---|---|---|
| * No. 1-25 is a formulation having the same buffer, pH, and stabilizer conditions as Tremfya^{®}. | | | | | |

### Preparation Example 2. Preparation of Formulations with Various Types of Stabilizers

Using guselkumab (CAS No. 1350289-85-8) as an anti-IL-23 antibody, an aqueous liquid formulation having the composition shown in Table 2 below was prepared.

Table 2 shows the compositions of 10 formulations (Nos. 2-1 to 2-8, and 2-10 to 2-11) prepared by applying various types of stabilizers currently used in approved biopharmaceuticals, and one formulation (No. 2-9) having the same buffer, pH, and stabilizer conditions as Tremfya^{®}.

The antibody concentration was included at 5 mg/ml for T_{agg} analysis.

**[Table 2]**

| No. | Antibody concentration (mg/ml) | Buffer | pH | Stabilizer | Surfactant |
|---|---|---|---|---|---|
| 2-1 | 5 | 20 mM histidine | 6.0 | 8.0% (w/v) sucrose | - |
| 2-2 | | | | 9.0% (w/v) trehalose | |
| 2-3 | | | | 3.13% (w/v) mannitol | |
| 2-4 | | | | 140 mM arginine | |
| 2-5 | | | | 135 mM lysine | |
| 2-6 | | | | 90 mM methionine | |
| 2-7 | | | | 250 mM glycine | |
| 2-8 | | | | 0.8% (w/v) NaCl | |
| 2-9 | 5 | 11 mM histidine | 5.8 | 7.9% (w/v) sucrose | - |
| 2-10 | 5 | 20 mM histidine | 6.0 | 4.7% (w/v) mannitol | - |
| 2-11 | | | | 150 mM methionine | |

| | | | | | |
|---|---|---|---|---|---|
| * No. 2-9 is a formulation having the same buffer, pH and stabilizer conditions as Tremfya^{®}. | | | | | |

### Preparation Example 3. Preparation of Formulations with Various Combinations of Buffers, pH, and Stabilizers

Using guselkumab (CAS No. 1350289-85-8) as an anti-IL-23 antibody, aqueous liquid formulations having the compositions shown in Table 3 below were prepared.

Table 3 shows the compositions of 12 formulations (Nos. 3-1 to 3-12) prepared by applying various buffer, pH, and stabilizer conditions, and one formulation (No. 3-13) having the same buffer, pH, and stabilizer conditions as Tremfya^{®}.

The concentration of the antibody was included at 100 mg/mL, which is the same as that of Tremfya^{®}.

**[Table 3]**

| No. | Antibody Concent ration (mg/ml) | Buffer | pH | Stabilizer 1 | Stabilizer 2 | Surfactant |
|---|---|---|---|---|---|---|
| 3-1 | 100 | 20 mM acetate | 5.8 | 9.5% (w/v) trehalose | - | 0.05% (w/v) PS80 |
| 3-2 | | 20 mM acetate | 5.8 | 4.7% (w/v) mannitol | - | |
| 3-3 | | 20 mM acetate | 5.8 | 280 mM glycine | - | |
| 3-4 | | 20 mM acetate | 5.8 | 8.5% (w/v) trehalose | 30 mM methionine | |
| 3-5 | | 20 mM acetate | 5.8 | 4.2% (w/v) mannitol | 30 mM methionine | |
| 3-6 | | 20 mM acetate | 5.8 | 250 mM glycine | 30 mM methionine | |
| 3-7 | | 20 mM histidine | 5.8 | 9.5% (w/v) trehalose | - | |
| 3-8 | | 20 mM histidine | 5.8 | 4.7% (w/v) mannitol | - | |
| 3-9 | | 20 mM histidine | 5.8 | 280 mM glycine | - | |
| 3-10 | | 20 mM histidine | 5.8 | 8.5% (w/v) trehalose | 30 mM methionine | |
| 3-11 | | 20 mM histidine | 5.8 | 4.2% (w/v) mannitol | 30 mM methionine | |
| 3-12 | | 20 mM histidine | 5.8 | 250 mM glycine | 30 mM methionine | |
| 3-13 | 100 | 11 mM histidine | 5.8 | 7.9% (w/v) sucrose | - | 0.05% (w/v) PS80 |

| | | | | | | |
|---|---|---|---|---|---|---|
| * No. 3-13 is a formulation having the same antibody concentration, buffer, pH, stabilizer, and surfactant conditions as Tremfya^{®}. | | | | | | |

### Preparation Example 4. Preparation of Formulations With or Without Surfactant

Using guselkumab (CAS No. 1350289-85-8) as an anti-IL-23 antibody, aqueous liquid formulations having the compositions shown in Table 4 below were prepared.

Table 4 shows the compositions of one formulation (No. 4-1) having the same buffer, pH, and stabilizer conditions as Tremfya^{®} but without a surfactant, and one formulation (No. 4-2) having the same buffer, pH, stabilizer, and surfactant conditions as Tremfya^{®}.

The concentration of the antibody was included at 100 mg/mL, which is the same as that of Tremfya^{®}.

**[Table 4]**

| No. | Antibody Concentra tion (mg/ml) | Buffer | pH | Stabilizer | Surfactant |
|---|---|---|---|---|---|
| 4-1 | 100 | 11 mM histidine | 5.8 | 7.9% (w/v) sucrose | - |
| 4-2 | | 11 mM histidine | 5.8 | 7.9% (w/v) sucrose | 0.05% (w/v) PS80 |

| | | | | | |
|---|---|---|---|---|---|
| * No. 4-2 is a formulation having the same antibody concentration, buffer, pH, stabilizer, and surfactant conditions as Tremfya^{®}. | | | | | |

### Preparation Example 5. Preparation of Formulations With or Without Amino Acids

Using guselkumab (CAS No. 1350289-85-8) as an anti-IL-23 antibody, aqueous liquid formulations having the compositions shown in Table 5 below were prepared.

Table 5 shows the compositions of 9 formulations (Nos. 5-1 to 5-9) prepared by applying various buffer, pH, and stabilizer conditions.

The concentration of the antibody was included at 100 mg/mL, which is the same as that of Tremfya^{®}.

**[Table 5]**

| No. | Antibo dy Conce ntratio n (mg/m l) | Buffer | pH | Stabilizer 1 | Stabilizer 2 | Surfactant |
|---|---|---|---|---|---|---|
| 5-1 | | 20 mM acetate | | 4.7% (w/v) mannitol | - | |
| 5-2 | | 20 mM acetate | | 4.7% (w/v) mannitol | 30 mM histidine | |
| 5-3 | | 20 mM acetate | | 4.7% (w/v) mannitol | 30 mM methionine | |
| 5-4 | 100 | 20 mM histidine | 6.0 | 4.7% (w/v) mannitol | - | |
| 5-5 | | 20 mM histidine | | 4.7% (w/v) mannitol | 30 mM histidine | 0.05% (w/v) PS80 |
| 5-6 | | 20 mM histidine | | 4.7% (w/v) mannitol | 30 mM methionine | |
| 5-7 | | 20 mM phosphate | | 4.7% (w/v) mannitol | - | |
| 5-8 | | 20 mM phosphate | | 4.7% (w/v) mannitol | 30 mM histidine | |
| 5-9 | | 20 mM phosphate | | 4.7% (w/v) mannitol | 30 mM methionine | |

### [Example]

### Example 1. Buffer/pH Screening

### 1.1 Screening for Optimal Combination of Buffer and pH through T_{agg} Measurement

The T_{agg} of the formulation prepared in Preparation Example 1 was measured. An increase in T_{agg} indicates that the temperature at which aggregation occurs has increased. Therefore, an increased T_{agg} indicates improved thermal stability.

Table 6 shows the results of measuring T_{agg} for 24 formulations in which buffer types and pH are combined in Preparation Example 1, and for one formulation (No. 1-25) having the same buffer and pH conditions as Tremfya^{®}.

**[Table 6]**

| No. | Buffer, pH | T_{agg} (°C) |
|---|---|---|
| 1-1 | 20 mM acetate, pH 4.0 | 78.99 |
| 1-2 | 20 mM acetate, pH 4.5 | 74.84 |
| 1-3 | 20 mM acetate, pH 5.0 | 74.52 |
| 1-4 | 20 mM acetate, pH 5.5 | 74.85 |
| 1-5 | 20 mM acetate, pH 6.0 | 74.89 |
| 1-6 | 20 mM histidine, pH 5.0 | 71.42 |
| 1-7 | 20 mM histidine, pH 5.5 | 73.29 |
| 1-8 | 20 mM histidine, pH 6.0 | 74.62 |
| 1-9 | 20 mM histidine, pH 6.5 | 74.88 |
| 1-10 | 20 mM histidine, pH 7.0 | 75.61 |
| 1-11 | 20 mM phosphate, pH 6.0 | 74.29 |
| 1-12 | 20 mM phosphate, pH 6.5 | 73.92 |
| 1-13 | 20 mM phosphate, pH 7.0 | 73.74 |
| 1-14 | 20 mM phosphate, pH 7.5 | 73.45 |
| 1-15 | 20 mM phosphate, pH 8.0 | 73.27 |
| 1-16 | 20 mM citrate, pH 4.5 | 63.09 |
| 1-17 | 20 mM citrate, pH 5.0 | 65.20 |
| 1-18 | 20 mM citrate, pH 5.5 | 69.46 |
| 1-19 | 20 mM citrate, pH 6.0 | 71.03 |
| 1-20 | 20 mM citrate, pH 6.5 | 71.97 |
| 1-21 | 20 mM succinate, pH 4.5 | 68.46 |
| 1-22 | 20 mM succinate, pH 5.0 | 69.38 |
| 1-23 | 20 mM succinate, pH 5.5 | 70.92 |
| 1-24 | 20 mM succinate, pH 6.0 | 72.19 |
| 1-25 | 11 mM histidine, pH 5.8 | 73.82 |

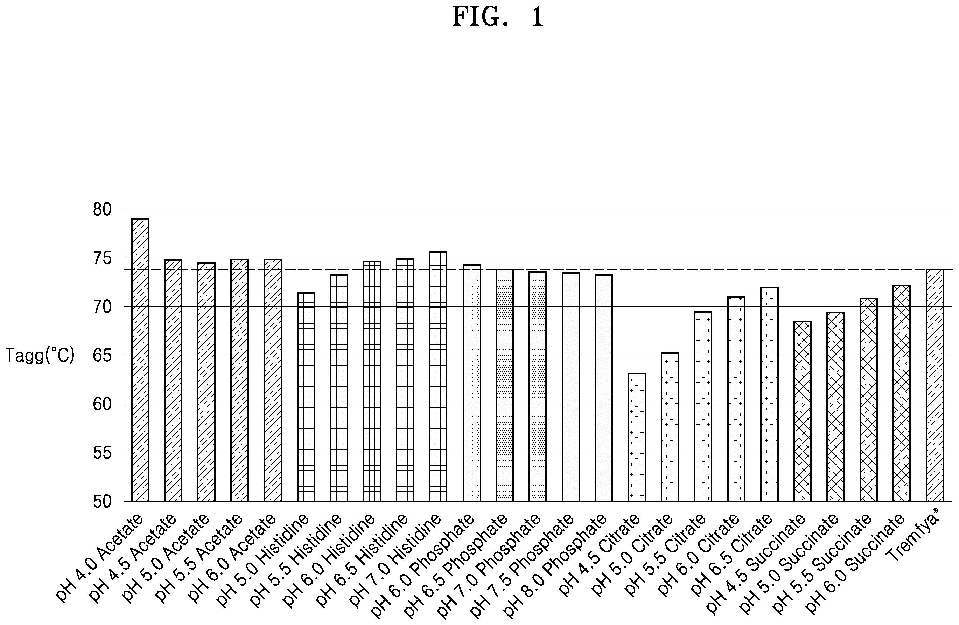

FIG. 1 is a graph showing the results of measuring T_{agg} for 24 formulations in which different buffer types and pH are combined in Preparation Example 1, and for one formulation having the same buffer and pH conditions as Tremfya^{®}.

As a result, as shown in Table 6 and FIG. 1, the acetate group and the histidine group generally exhibited higher T_{agg} values compared to Formulation No. 1-25, which has the same 11 mM histidine buffer and pH 5.8 conditions as Tremfya^{®}, confirming improved thermal stability.

### 1.2 Screening for Optimal Combination of Buffer and pH through K_{D} Measurement

The K_{D} of the formulations prepared in Preparation Example 1 was measured. Attractive interaction between proteins is a major cause of protein aggregation. An increase in K_{D} indicates a decrease in the attractive interaction between proteins. Therefore, an increased K_{D} indicates improved stability.

Table 7 shows the results of measuring K_{D} for the 24 formulations in which buffer types and pH are combined in Preparation Example 1, and for one formulation (No. 1-25) having the same buffer and pH conditions as Tremfya^{®}.

**[Table 7]**

| No. | Buffer, pH | K_{D} (mL/g) |
|---|---|---|
| 1-1 | 20 mM acetate, pH 4.0 | 160.2 |
| 1-2 | 20 mM acetate, pH 4.5 | 66.6 |
| 1-3 | 20 mM acetate, pH 5.0 | 30.9 |
| 1-4 | 20 mM acetate, pH 5.5 | 17.1 |
| 1-5 | 20 mM acetate, pH 6.0 | 11.0 |
| 1-6 | 20 mM histidine, pH 5.0 | 16.7 |
| 1-7 | 20 mM histidine, pH 5.5 | 16.4 |
| 1-8 | 20 mM histidine, pH 6.0 | 21.1 |
| 1-9 | 20 mM histidine, pH 6.5 | 29.4 |
| 1-10 | 20 mM histidine, pH 7.0 | 44.6 |
| 1-11 | 20 mM phosphate, pH 6.0 | 2.0 |
| 1-12 | 20 mM phosphate, pH 6.5 | -4.6 |
| 1-13 | 20 mM phosphate, pH 7.0 | -10.0 |
| 1-14 | 20 mM phosphate, pH 7.5 | -12.3 |
| 1-15 | 20 mM phosphate, pH 8.0 | -13.5 |
| 1-16 | 20 mM citrate, pH 4.5 | -3.4 |
| 1-17 | 20 mM citrate, pH 5.0 | -7.2 |
| 1-18 | 20 mM citrate, pH 5.5 | -10.6 |
| 1-19 | 20 mM citrate, pH 6.0 | -11.7 |
| 1-20 | 20 mM citrate, pH 6.5 | -12.8 |
| 1-21 | 20 mM succinate, pH 4.5 | 20.6 |
| 1-22 | 20 mM succinate, pH 5.0 | 6.7 |
| 1-23 | 20 mM succinate, pH 5.5 | -0.8 |
| 1-24 | 20 mM succinate, pH 6.0 | -3.6 |
| 1-25 | 11 mM histidine, pH 5.8 | 19.9 |

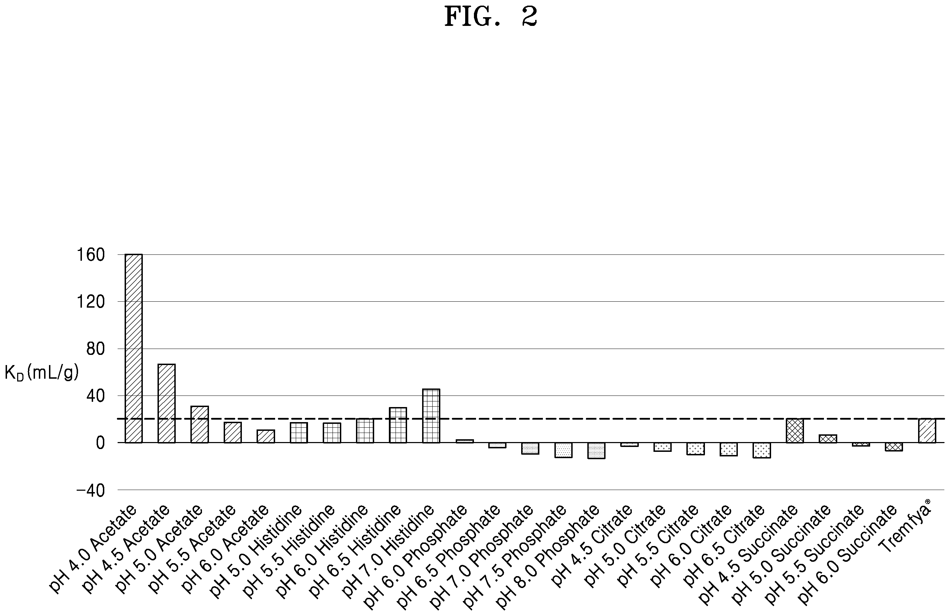

FIG. 2 is a graph showing the results of measuring K_{D} for 24 formulations in which different buffer types and pH are combined in Preparation Example 1, and for one formulation having the same buffer and pH conditions as Tremfya^{®}.

As a result, as shown in FIG. 2 and Table 7, the acetate group and the histidine group generally exhibited increased K_{D} values compared to Formulation No. 1-25, which has the same 11 mM histidine buffer and pH 5.8 conditions as Tremfya^{®}, confirming that stability was improved.

### 1.3 SEC Analysis Results

The %HMW (High-molecular weight %) for the formulations prepared in Preparation Example 1 was confirmed through SEC analysis. Since the presence of HMW indicates protein aggregation, a smaller %HMW value indicates improved stability.

Table 8 shows the results of measuring %HMW (initial) for 24 formulations in which buffer types and pH are combined in Preparation Example 1, and for one formulation (No. 1-25) having the same buffer and pH conditions as Tremfya^{®}.

**[Table 8]**

| No. | Buffer, pH | %HMW (initial) |
|---|---|---|
| 1-1 | 20 mM acetate, pH 4.0 | 0.6 |
| 1-2 | 20 mM acetate, pH 4.5 | 0.6 |
| 1-3 | 20 mM acetate, pH 5.0 | 0.6 |
| 1-4 | 20 mM acetate, pH 5.5 | 0.7 |
| 1-5 | 20 mM acetate, pH 6.0 | 0.8 |
| 1-6 | 20 mM histidine, pH 5.0 | 0.6 |
| 1-7 | 20 mM histidine, pH 5.5 | 0.7 |
| 1-8 | 20 mM histidine, pH 6.0 | 0.7 |
| 1-9 | 20 mM histidine, pH 6.5 | 0.7 |
| 1-10 | 20 mM histidine, pH 7.0 | 0.8 |
| 1-11 | 20 mM phosphate, pH 6.0 | 0.9 |
| 1-12 | 20 mM phosphate, pH 6.5 | 1.0 |
| 1-13 | 20 mM phosphate, pH 7.0 | 1.2 |
| 1-14 | 20 mM phosphate, pH 7.5 | 1.4 |
| 1-15 | 20 mM phosphate, pH 8.0 | 1.9 |
| 1-16 | 20 mM citrate, pH 4.5 | 0.7 |
| 1-17 | 20 mM citrate, pH 5.0 | 0.7 |
| 1-18 | 20 mM citrate, pH 5.5 | 0.8 |
| 1-19 | 20 mM citrate, pH 6.0 | 0.9 |
| 1-20 | 20 mM citrate, pH 6.5 | 1.0 |
| 1-21 | 20 mM succinate, pH 4.5 | 0.6 |
| 1-22 | 20 mM succinate, pH 5.0 | 0.7 |
| 1-23 | 20 mM succinate, pH 5.5 | 0.8 |
| 1-24 | 20 mM succinate, pH 6.0 | 0.9 |
| 1-25 | 11 mM histidine, pH 5.8 | 0.7 |

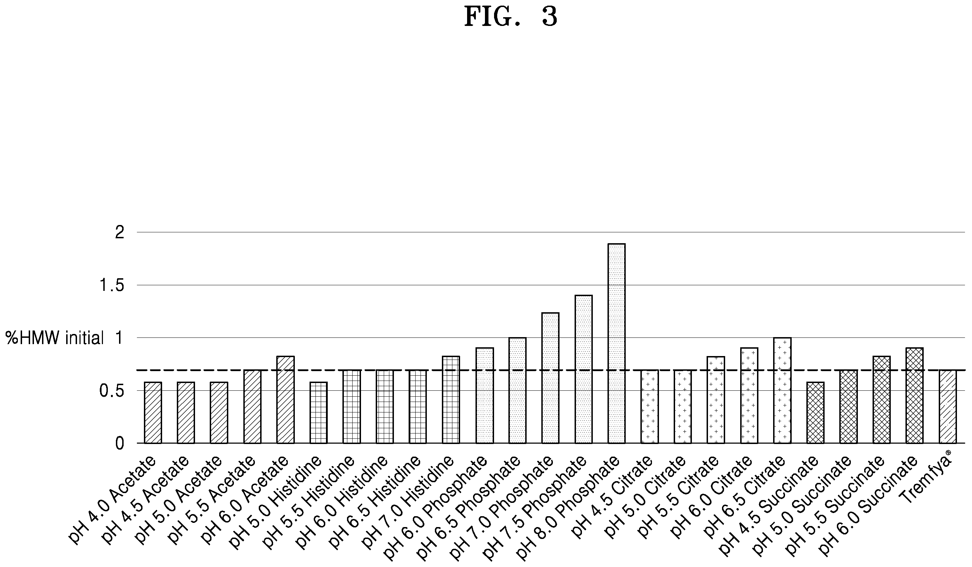

FIG. 3 is a graph showing the results of measuring %HMW (initial) through SEC analysis for 24 formulations in which different buffer types and pH are combined in Preparation Example 1, and for one formulation having the same buffer and pH conditions as Tremfya^{®}.

As a result, as shown in Table 8 and FIG. 3, it was confirmed that the %HMW values were generally lower in the acetate group and the histidine group compared to other buffer groups.

### 1.4 Data Analysis

Table 9 below shows the results of screening for the optimal buffer and pH by integrating the results of Examples 1.1 to 1.3 above. As a result, as shown in Table 9 below, it was confirmed that stability was generally higher in the acetate group and the histidine group compared to the phosphate group, citrate group, and succinate group. Consequently, two types of buffers, acetate and histidine, were selected as candidates for subsequent experiments.

**[Table 9]**

| No. | Buffer, pH | T_{agg} (°C) | K_{D} (mL/g) | %HMW (initial) |
|---|---|---|---|---|
| 1-1 | 20 mM acetate, pH 4.0 | 78.99 | 160.2 | 0.6 |
| 1-2 | 20 mM acetate, pH 4.5 | 74.84 | 66.6 | 0.6 |
| 1-3 | 20 mM acetate, pH 5.0 | 74.52 | 30.9 | 0.6 |
| 1-4 | 20 mM acetate, pH 5.5 | 74.85 | 17.1 | 0.7 |
| 1-5 | 20 mM acetate, pH 6.0 | 74.89 | 11.0 | 0.8 |
| 1-6 | 20 mM histidine, pH 5.0 | 71.42 | 16.7 | 0.6 |
| 1-7 | 20 mM histidine, pH 5.5 | 73.29 | 16.4 | 0.7 |
| 1-8 | 20 mM histidine, pH 6.0 | 74.62 | 21.1 | 0.7 |
| 1-9 | 20 mM histidine, pH 6.5 | 74.88 | 29.4 | 0.7 |
| 1-10 | 20 mM histidine, pH 7.0 | 75.61 | 44.6 | 0.8 |
| 1-11 | 20 mM phosphate, pH 6.0 | 74.29 | 2.0 | 0.9 |
| 1-12 | 20 mM phosphate, pH 6.5 | 73.92 | -4.6 | 1.0 |
| 1-13 | 20 mM phosphate, pH 7.0 | 73.74 | -10.0 | 1.2 |
| 1-14 | 20 mM phosphate, pH 7.5 | 73.45 | -12.3 | 1.4 |
| 1-15 | 20 mM phosphate, pH 8.0 | 73.27 | -13.5 | 1.9 |
| 1-16 | 20 mM citrate, pH 4.5 | 63.09 | -3.4 | 0.7 |
| 1-17 | 20 mM citrate, pH 5.0 | 65.20 | -7.2 | 0.7 |
| 1-18 | 20 mM citrate, pH 5.5 | 69.46 | -10.6 | 0.8 |
| 1-19 | 20 mM citrate, pH 6.0 | 71.03 | -11.7 | 0.9 |
| 1-20 | 20 mM citrate, pH 6.5 | 71.97 | -12.8 | 1.0 |
| 1-21 | 20 mM succinate, pH 4.5 | 68.46 | 20.6 | 0.6 |
| 1-22 | 20 mM succinate, pH 5.0 | 69.38 | 6.7 | 0.7 |
| 1-23 | 20 mM succinate, pH 5.5 | 70.92 | -0.8 | 0.8 |
| 1-24 | 20 mM succinate, pH 6.0 | 72.19 | -3.6 | 0.9 |
| 1-25 | 11 mM histidine, pH 5.8 | 73.82 | 19.9 | 0.7 |

### Example 2. Stabilizer Screening

### 2.1 Screening for Optimal Stabilizer through T_{agg} Measurement

The T_{agg} of the formulations prepared in Preparation Example 2 was measured.

Table 10 shows the results of measuring T_{agg} for 10 formulations having various types of stabilizers in Preparation Example 2, and for one formulation having the same stabilizer conditions as Tremfya^{®}.

**[Table 10]**

| No. | Stabilizer | T_{agg} (°C) |
|---|---|---|
| 2-1 | 8.0% (w/v) sucrose | 73.39 |
| 2-2 | 9.0% (w/v) trehalose | 74.70 |
| 2-3 | 3.13% (w/v) mannitol | 75.66 |
| 2-4 | 140 mM arginine | 69.46 |
| 2-5 | 135 mM lysine | 69.97 |
| 2-6 | 90 mM methionine | 74.13 |
| 2-7 | 250 mM glycine | 75.10 |
| 2-8 | 0.8% (w/v) NaCl | 69.76 |
| 2-9 | 7.9% (w/v) sucrose | 73.82 |
| 2-10 | 4.7% (w/v) mannitol | 75.71 |
| 2-11 | 150 mM methionine | 74.34 |

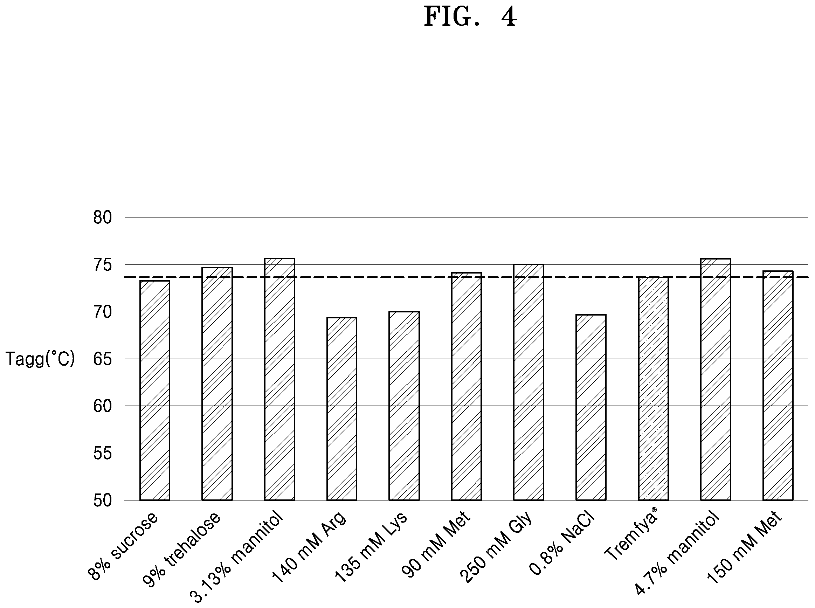

FIG. 4 is a graph showing the results of measuring T_{agg} for 10 formulations having various types of stabilizers in Preparation Example 2, and for one formulation having the same stabilizer conditions as Tremfya^{®}.

As a result, as shown in Table 10 and FIG. 4, it was confirmed that trehalose, mannitol, methionine, and glycine exhibited increased T_{agg} values compared to Formulation No. 2-9, which has the same 7.9% (w/v) sucrose stabilizer conditions as Tremfya^{®}. Accordingly, four types of stabilizers, glycine, mannitol, methionine, and trehalose, were selected as candidates for subsequent experiments.

### Example 3. Screening for Combinations of Buffer, pH, and Stabilizer

### 3.1 SEC Analysis Results under Thermal Stress Conditions (25 °C, 5 °C)

To test the stability of formulations containing various combinations of buffers, pH, and stabilizers prepared in Preparation Example 3, the samples of Table 3 prepared in Preparation Example 3 were exposed to thermal stress conditions of 25 °C or 5 °C for 4 weeks. Subsequently, the %HMW (High-molecular weight %) and its rate of change (Δ%HMW) over storage time were confirmed through SEC analysis. The results are shown in Tables 11 and 12 below. Since the presence of HMW indicates protein aggregation, a smaller Δ%HMW value indicates improved stability.

**[Table 11]**

| No. | Formulation | %HMW (25 °C) | | | %ΔHMW (25 °C) |
|---|---|---|---|---|---|
| | | 0 | 2 | 4 | 4 |
| 3-1 | 20 mM Ace, 9.5% Tre | 0.86 | 1.34 | 1.43 | 0.57 |
| 3-2 | 20 mM Ace, 4.7% Man | 0.88 | 1.35 | 1.43 | 0.55 |
| 3-3 | 20 mM Ace, 280 mM Gly | 0.80 | 1.15 | 1.29 | 0.49 |
| 3-4 | 20 mM Ace, 8.5% Tre, 30 mM Met | 0.82 | 1.16 | 1.26 | 0.44 |
| 3-5 | 20 mM Ace, 4.2% Man, 30 mM Met | 0.83 | 1.17 | 1.27 | 0.44 |
| 3-6 | 20 mM Ace, 250 mM Gly, 30 mM Met | 0.77 | 1.09 | 1.17 | 0.40 |
| 3-7 | 20 mM His, 9.5% Tre | 0.81 | 1.10 | 1.17 | 0.36 |
| 3-8 | 20 mM His, 4.7% Man | 0.83 | 1.11 | 1.18 | 0.35 |
| 3-9 | 20 mM His, 280 mM Gly | 0.80 | 1.06 | 1.13 | 0.33 |
| 3-10 | 20 mM His, 8.5% Tre, 30 mM Met | 0.79 | 1.06 | 1.12 | 0.33 |
| 3-11 | 20 mM His, 4.2% Man, 30 mM Met | 0.79 | 1.06 | 1.10 | 0.31 |
| 3-12 | 20 mM His, 250 mM Gly, 30 mM Met | 0.77 | 1.01 | 1.05 | 0.28 |
| 3-13 | 11 mM His, 7.9% sucrose (Tremfya^{®} formulation) | 0.89 (N=3, SD: 0.01) | 1.29 (N=3, SD: 0.01) | 1.38 (N=3, SD: 0.01) | 0.49 (N=3, SD: 0.01) |

**[Table 12]**

| No. | Formulation | | %HMW (5 °C) | %ΔHMW (5 °C) |
|---|---|---|---|---|
| | | 0 | 4 | 4 |
| 3-1 | 20 mM Ace, 9.5% Tre | 0.86 | 1.07 | 0.21 |
| 3-2 | 20 mM Ace, 4.7% Man | 0.88 | 1.06 | 0.18 |
| 3-3 | 20 mM Ace, 280 mM Gly | 0.80 | 0.90 | 0.10 |
| 3-4 | 20 mM Ace, 8.5% Tre, 30 mM Met | 0.82 | 0.94 | 0.12 |
| 3-5 | 20 mM Ace, 4.2% Man, 30 mM Met | 0.83 | 0.94 | 0.11 |
| 3-6 | 20 mM Ace, 250 mM Gly, 30 mM Met | 0.77 | 0.87 | 0.10 |
| 3-7 | 20 mM His, 9.5% Tre | 0.81 | 0.93 | 0.12 |
| 3-8 | 20 mM His, 4.7% Man | 0.83 | 0.95 | 0.12 |
| 3-9 | 20 mM His, 280 mM Gly | 0.80 | 0.92 | 0.12 |
| 3-10 | 20 mM His, 8.5% Tre, 30 mM Met | 0.79 | 0.91 | 0.12 |
| 3-11 | 20 mM His, 4.2% Man, 30 mM Met | 0.79 | 0.91 | 0.12 |
| 3-12 | 20 mM His, 250 mM Gly, 30 mM Met | 0.77 | 0.87 | 0.10 |
| 3-13 | 11 mM His, 7.9% sucrose (Tremfya^{®} formulation) | 0.89 (N=3, SD: 0.01) | 1.07 (N=3, SD: 0.01) | 0.18 (N=3, SD: 0.01) |

As a result, as shown in Table 11, it was confirmed that at 25 °C, Formulations Nos. 3-3 to 3-12 exhibited %ΔHMW values equivalent to or lower than those of the Tremfya^{®} formulation (control), indicating improved stability.

Additionally, as shown in Table 12, it was confirmed that at 5 °C, Formulations Nos. 3-2 to 3-12 exhibited %ΔHMW values equivalent to or lower than those of the Tremfya^{®} formulation (control), indicating improved stability.

### 3.2 SEC Analysis Results under Freeze-Thaw Stress Conditions

To test the stability of the formulations comprising various combinations of buffers, pH, and stabilizers prepared in Preparation Example 3, the samples of Table 3 were subjected to freeze-thaw stress under conditions of 5 cycles of freezing and thawing at - 70 °C±10 °C. Subsequently, the %HMW (High-molecular weight %) and its rate of change (Δ%HMW) before and after the 5 cycles were confirmed through SEC analysis. The results are shown in Table 13 below.

**[Table 13]**

| No. | Formulation | %HMW (F/T) | | %ΔHMW (F/T) |
|---|---|---|---|---|
| | | Initial | 5 cycle | 5 cycle |
| 3-1 | 20 mM Ace, 9.5% Tre | 0.9 | 0.9 | 0.1 |
| 3-2 | 20 mM Ace, 4.7% Man | 0.9 | 0.9 | 0.0 |
| 3-3 | 20 mM Ace, 280 mM Gly | 0.8 | 0.9 | 0.1 |
| 3-4 | 20 mM Ace, 8.5% Tre, 30 mM Met | 0.8 | 0.9 | 0.1 |
| 3-5 | 20 mM Ace, 4.2% Man, 30 mM Met | 0.8 | 0.8 | 0.0 |
| 3-6 | 20 mM Ace, 250 mM Gly, 30 mM Met | 0.8 | 0.8 | 0.1 |
| 3-7 | 20 mM His, 9.5% Tre | 0.8 | 0.9 | 0.0 |
| 3-8 | 20 mM His, 4.7% Man | 0.8 | 0.9 | 0.0 |
| 3-9 | 20 mM His, 280 mM Gly | 0.8 | 0.9 | 0.1 |
| 3-10 | 20 mM His, 8.5% Tre, 30 mM Met | 0.8 | 0.9 | 0.1 |
| 3-11 | 20 mM His, 4.2% Man, 30 mM Met | 0.8 | 0.9 | 0.1 |
| 3-12 | 20 mM His, 250 mM Gly, 30 mM Met | 0.8 | 0.8 | 0.0 |
| 3-13 | 11 mM His, 7.9% sucrose (Tremfya^{®} formulation) | 0.9 (N=3, SD= 0.0) | 0.9 (N=3, SD= 0.0) | 0.1 (N=3, SD= 0.0) |

As a result, as shown in Table 13, it was confirmed that all tested formulations Nos. 3-1 to 3-12 exhibited %ΔHMW values at a level equivalent to that of the Tremfya^{®} formulation (control).

### 3.3 SEC Analysis Results under Agitation Stress Conditions

To test the stability of formulations containing various combinations of buffer/pH/stabilizer prepared in Preparation Example 3, stirring stress was applied to the samples of Table 3 prepared in Preparation Example 3 under the condition of stirring at 400 rpm for 72 hours, and then the %HMW (High-molecular weight %) and its change rate (Δ%HMW) before and after stirring were confirmed through SEC analysis. The results are shown in Table 14 below.

**[Table 14]**

| No. | Formulation | %HMW (AG) | | %ΔHMW (AG) |
|---|---|---|---|---|
| | | Initial | 400 rpm | 400 rpm |
| 3-1 | 20 mM Ace, 9.5% Tre | 0.9 | 1.1 | 0.2 |
| 3-2 | 20 mM Ace, 4.7% Man | 0.9 | 1.1 | 0.2 |
| 3-3 | 20 mM Ace, 280 mM Gly | 0.8 | 0.9 | 0.1 |
| 3-4 | 20 mM Ace, 8.5% Tre, 30 mM Met | 0.8 | 1.0 | 0.1 |
| 3-5 | 20 mM Ace, 4.2% Man, 30 mM Met | 0.8 | 1.0 | 0.1 |
| 3-6 | 20 mM Ace, 250 mM Gly, 30 mM Met | 0.8 | 0.9 | 0.1 |
| 3-7 | 20 mM His, 9.5% Tre | 0.8 | 0.9 | 0.1 |
| 3-8 | 20 mM His, 4.7% Man | 0.8 | 0.9 | 0.1 |
| 3-9 | 20 mM His, 280 mM Gly | 0.8 | 0.9 | 0.1 |
| 3-10 | 20 mM His, 8.5% Tre, 30 mM Met | 0.8 | 0.9 | 0.1 |
| 3-11 | 20 mM His, 4.2% Man, 30 mM Met | 0.8 | 0.9 | 0.1 |
| 3-12 | 20mM His, 250mM Gly, 30mM Met | 0.8 | 0.9 | 0.1 |
| 3-13 | 11 mM His, 7.9% sucrose (Tremfya^{®} formulation) | 0.9 (N=3, SD= 0.0) | 1.1 (N=3, SD= 0.0) | 0.2 (N=3, SD= 0.0) |

As a result, as shown in Table 14, it was confirmed that all tested formulations Nos. 3-1 to 3-12 exhibited %ΔHMW values at a level equivalent to that of the Tremfya^{®} formulation (control).

### 3.4 Viscosity Evaluation

To test the stability of the formulations comprising various combinations of buffers, pH, and stabilizers prepared in Preparation Example 3, the viscosity of the samples in Table 3 prepared in Preparation Example 3 was measured. The results are shown in Table 15 below.

**[Table 15]**

| No. | Formulation | viscosity (cP) |
|---|---|---|
| 3-1 | 20 mM Ace, 9.5% Tre | 3.7 |
| 3-2 | 20 mM Ace, 4.7% Man | 3.3 |
| 3-3 | 20 mM Ace, 280 mM Gly | 3.0 |
| 3-4 | 20 mM Ace, 8.5% Tre, 30 mM Met | 3.6 |
| 3-5 | 20 mM Ace, 4.2% Man, 30 mM Met | 3.3 |
| 3-6 | 20 mM Ace, 250 mM Gly, 30 mM Met | 3.1 |
| 3-7 | 20 mM His, 9.5% Tre | 3.7 |
| 3-8 | 20 mM His, 4.7% Man | 3.4 |
| 3-9 | 20 mM His, 280 mM Gly | 3.1 |
| 3-10 | 20 mM His, 8.5% Tre, 30 mM Met | 3.8 |
| 3-11 | 20 mM His, 4.2% Man, 30 mM Met | 3.4 |
| 3-12 | 20 mM His, 250 mM Gly, 30 mM Met | 3.1 |
| 3-13 | 11 mM His, 7.9% sucrose (Tremfya^{®} formulation) | 3.8 (N=3, SD=0.1) |

As a result, as shown in Table 15, it was confirmed that all tested formulations Nos. 3-1 to 3-12 exhibited viscosity values equivalent to or lower than those of the Tremfya^{®} formulation (control), indicating improved stability. In particular, compared to the group containing trehalose as a stabilizer, the group containing mannitol as a stabilizer showed improved viscosity in the range of approximately 3.3 to 3.4 cP, and the group containing glycine as a stabilizer showed significantly improved viscosity in the range of approximately 3.0 to 3.1 cP.

### 3.5 Data Analysis and Analysis of Amino Acid Ratio to Antibody

Table 16 below summarizes the results of Examples 3.1 to 3.4, excluding the SEC analysis results under freeze-thaw stress or agitation stress conditions (Examples 3.2 and 3.3), where no significant differences were observed between the experimental groups and the control group.

**[Table 16]**

| No. | Formulation | %ΔHMW (25 °C) | %ΔHMW (5 °C) | viscosity (cP) |
|---|---|---|---|---|
| 3-1 | 20 mM Ace, 9.5% Tre | 0.57 | 0.21 | 3.7 |
| 3-2 | 20 mM Ace, 4.7% Man | 0.55 | 0.18 | 3.3 |
| 3-3 | 20 mM Ace, 280 mM Gly | 0.49 | 0.10 | 3.0 |
| 3-4 | 20 mM Ace, 8.5% Tre, 30 mM Met | 0.44 | 0.12 | 3.6 |
| 3-5 | 20 mM Ace, 4.2% Man, 30 mM Met | 0.44 | 0.11 | 3.3 |
| 3-6 | 20 mM Ace, 250 mM Gly, 30 mM Met | 0.40 | 0.10 | 3.1 |
| 3-7 | 20 mM His, 9.5% Tre | 0.36 | 0.12 | 3.7 |
| 3-8 | 20 mM His, 4.7% Man | 0.35 | 0.12 | 3.4 |
| 3-9 | 20 mM His, 280 mM Gly | 0.33 | 0.12 | 3.1 |
| 3-10 | 20 mM His, 8.5% Tre, 30 mM Met | 0.33 | 0.12 | 3.8 |
| 3-11 | 20 mM His, 4.2% Man, 30 mM Met | 0.31 | 0.12 | 3.4 |
| 3-12 | 20 mM His, 250 mM Gly, 30 mM Met | 0.28 | 0.10 | 3.1 |
| 3-13 | 11 mM His, 7.9% sucrose (Tremfya^{®} formulation) | 0.49 (N=3, SD: 0.01) | 0.18 (N=3, SD: 0.01) | 3.8 (N=3, SD= 0.1) |

As a result, as shown in Table 16, it was confirmed that Formulations Nos. 3-3 to 3-12 all exhibited superior stability compared to the Tremfya^{®} formulation (No. 3-13), which served as a control. In particular, it was confirmed that overall stability was excellent when histidine was used as a buffer and mannitol or glycine was used as a stabilizer. Furthermore, it was confirmed that the addition of methionine as a stabilizer further improved stability (Nos. 3-10 to 3-12).

In addition, for Formulations Nos. 3-1 to 3-12, the molar ratio of amino acids to the antibody (mAb:a.a.) was measured. The results are shown in Table 17 below.

**[Table 17]**

| No. | Formulation | mol/L | | | | | mAb: aa |
|---|---|---|---|---|---|---|---|
| | | Guselk umab | Histidin e | Glyci ne | Metioni ne | Total aa | |
| 3-1 | 20 mM Ace, 9.5% Tre | 0.0007 | | | | 0 | 1:0 |
| 3-2 | 20 mM Ace, 4.7% Man | 0.0007 | | | | 0 | 1:0 |
| 3-3 | 20 mM Ace, 280 mM Gly | 0.0007 | | 0.28 | | 0.28 | 1:411 |
| 3-4 | 20mM Ace, 8.5% Tre, 30mM Met | 0.0007 | | | 0.03 | 0.03 | 1:44 |
| 3-5 | 20mM Ace, 4.2% Man, 30mM Met | 0.0007 | | | 0.03 | 0.03 | 1:44 |
| 3-6 | 20mM Ace, 250mM Gly, 30mM Met | 0.0007 | | 0.25 | 0.03 | 0.28 | 1:411 |
| 3-7 | 20 mM His, 9.5% Tre | 0.0007 | 0.02 | | | 0.02 | 1:29 |
| 3-8 | 20 mM His, 4.7% Man | 0.0007 | 0.02 | | | 0.02 | 1:29 |
| 3-9 | 20 mM His, 280 mM Gly | 0.0007 | 0.02 | 0.28 | | 0.3 | 1:440 |
| 3-10 | 20mM His, 8.5% Tre, 30mM Met | 0.0007 | 0.02 | | 0.03 | 0.05 | 1:73 |
| 3-11 | 20mM His, 4.2% Man, 30mM Met | 0.0007 | 0.02 | | 0.03 | 0.05 | 1:73 |
| 3-12 | 20 mM His, 250 mM Gly, 30 mM Met | 0.0007 | 0.02 | 0.25 | 0.03 | 0.3 | 1:440 |
| 3-13 | 11 mM His, 7.9% sucrose (Tremfya^{®} formulation) | 0.0007 | 0.011 | | | 0.011 | 1:16 |

As a result, as shown in Table 17, while the Tremfya^{®} formulation (No. 3-13), used as a control, contained 16 mol of amino acids per 1 mol of the antibody, Formulations Nos. 3-3 to 3-12, which were confirmed to have superior stability compared to the Tremfya^{®} formulation, were found to contain 29 mol or more of amino acids per 1 mol of the antibody, confirming a relatively higher amino acid ratio.

### Example 4. Analysis of the Effect of Surfactant Inclusion

To test the stability of formulations depending on the inclusion of a surfactant, SEC analysis was performed on the samples of Table 4 prepared in Preparation Example 4 under thermal stress conditions (40°C, 25°C), freeze-thaw stress conditions, and agitation stress conditions in the same manner as in Example 3. The results are shown in Tables 18 to 21 below.

**[Table 18]**

| No. | Formulation | %HMW (40 °C) | | | %ΔHMW (40 °C) |
|---|---|---|---|---|---|
| | | 0 | 2 | 4 | 4 |
| 4-1 | 11 mM His, 7.9% sucrose (surfactant-free) | 1.5 | 2.0 | 2.3 | 0.8 |
| 4-2 | 11 mM His, 7.9% sucrose, 0.05% PS80 (Tremfya^{®} formulation) | 1.5 (N=3, SD: 0.0) | 2.0 (N=3, SD: 0.0) | 2.3 (N=3, SD: 0.0) | 0.9 (N=3, SD: 0.0) |

**[Table 19]**

| No. | Formulation | %HMW (25 °C) | | | %ΔHMW (25 °C) |
|---|---|---|---|---|---|
| | | 0 | 2 | 4 | 4 |
| 4-1 | 11 mM His, 7.9% sucrose (surfactant-free) | 1.5 | 1.6 | 1.7 | 0.2 |
| 4-2 | 11 mM His, 7.9% sucrose, 0.05% PS80 (Tremfya^{®} formulation) | 1.5 (N=3, SD: 0.0) | 1.7 (N=3, SD: 0.1) | 1.7 (N=3, SD: 0.0) | 0.2 (N=3, SD: 0.0) |

**[Table 20]**

| No. | Formulation | %HMW (F/T) | | %ΔHMW (F/T) |
|---|---|---|---|---|
| | | Initial | 5 cycle | 5 cycle |
| 4-1 | 11 mM His, 7.9% sucrose (surfactant-free) | 1.5 | 1.5 | 0.0 |
| 4-2 | 11 mM His, 7.9% sucrose, 0.05% PS80 (Tremfya^{®} formulation) | 1.5 (N=3, SD: 0.0) | 1.5 (N=3, SD: 0.0) | 0.0 (N=3, SD: 0.0) |

**[Table 21]**

| No. | Formulation | %HMW (AG) | | %ΔHMW (AG) |
|---|---|---|---|---|
| | | Initial | 400 rpm | 400 rpm |
| 4-1 | 11 mM His, 7.9% sucrose (surfactant-free) | 1.5 | 1.5 | 0.0 |
| 4-2 | 11 mM His, 7.9% sucrose, 0.05% PS80 (Tremfya^{®} formulation) | 1.5 (N=3, SD: 0.0) | 1.5 (N=3, SD: 0.0) | 0.0 (N=3, SD: 0.0) |

As a result, as shown in Tables 18 to 21, Formulation No. 4-1, which does not contain a surfactant, and Formulation No. 4-2, which contains a surfactant, exhibited equivalent levels of ΔHMW values. This confirms that in the stability of an aqueous liquid formulation comprising guselkumab, the effect of the presence or absence of a surfactant is not significant.

### Example 5. Analysis of the Effect of Amino Acid Inclusion and Analysis of Amino Acid Ratio to Antibody

To test the stability of formulations depending on the inclusion of amino acids, SEC analysis was performed on the samples of Table 5 prepared in Preparation Example 5 under thermal stress conditions (40 °C, 25 °C) in the same manner as in Example 3. The results are shown in Tables 22 and 23 below.

**[Table 22]**

| No. | Formulation | %HMW (40 °C) | | | %ΔHMW (40 °C) |
|---|---|---|---|---|---|
| | | 0 | 1 | 2 | 2 |
| 5-1 | 20 mM Ace, 4.7% Man (amino acid-free) | 1.5 | 2.0 | 2.4 | 0.9 |
| 5-2 | 20 mM Ace, 4.7% Man, 30 mM His | 1.3 | 1.5 | 1.7 | 0.5 |
| 5-3 | 20 mM Ace, 4.7% Man, 30 mM Met | 1.3 | 1.6 | 1.9 | 0.6 |
| 5-4 | 20 mM His, 4.7% Man | 1.3 | 1.5 | 1.8 | 0.4 |
| 5-5 | 20 mM His, 4.7% Man, 30 mM His | 1.2 | 1.4 | 1.6 | 0.4 |
| 5-6 | 20 mM His, 4.7% Man, 30 mM Met | 1.3 | 1.3 | 1.5 | 0.3 |
| 5-7 | 20 mM Pho, 4.7% Man(amino acid-free) | 1.6 | 2.5 | 3.0 | 1.4 |
| 5-8 | 20 mM Pho, 4.7% Man, 30 mM His | 1.2 | 1.6 | 1.8 | 0.6 |
| 5-9 | 20 mM Pho, 4.7% Man, 30 mM Met | 1.4 | 1.9 | 2.3 | 0.9 |

**[Table 23]**

| No. | Formulation | %HMW (25 °C) | | | %ΔHMW (25 °C) |
|---|---|---|---|---|---|
| | | 0 | 2 | 4 | 4 |
| 5-1 | 20 mM Ace, 4.7% Man (amino acid-free) | 1.5 | 1.8 | 1.9 | 0.4 |
| 5-2 | 20 mM Ace, 4.7% Man, 30 mM His | 1.3 | 1.3 | 1.5 | 0.2 |
| 5-3 | 20 mM Ace, 4.7% Man, 30 mM Met | 1.3 | 1.4 | 1.5 | 0.2 |
| 5-4 | 20 mM His, 4.7% Man | 1.3 | 1.4 | 1.5 | 0.2 |
| 5-5 | 20 mM His, 4.7% Man, 30 mM His | 1.2 | 1.3 | 1.4 | 0.1 |
| 5-6 | 20 mM His, 4.7% Man, 30 mM Met | 1.3 | 1.3 | 1.4 | 0.1 |
| 5-7 | 20 mM Pho, 4.7% Man(amino acid-free) | 1.6 | 2.1 | 2.3 | 0.7 |
| 5-8 | 20 mM Pho, 4.7% Man, 30 mM His | 1.2 | 1.3 | 1.5 | 0.3 |
| 5-9 | 20 mM Pho, 4.7% Man, 30 mM Met | 1.4 | 1.6 | 1.8 | 0.4 |

As a result, as shown in Tables 22 to 23, in all buffer groups, the ΔHMW values of Formulations Nos. 5-2, 5-3, 5-4, 5-5, 5-6, 5-8, and 5-9, which contain amino acids, were lower than those of Formulations Nos. 5-1 and 5-7, which do not contain amino acids. This confirms that in the stability of an aqueous liquid formulation including guselkumab, stability is improved when amino acids are included.

In addition, for Formulations Nos. 5-1 to 5-9, the molar ratio of amino acids to the antibody (mAb:a.a.) was measured. The results are shown in Table 24 below.

**[Table 24]**

| No. | Formulation | mol/ml | | | | | mAb: aa |
|---|---|---|---|---|---|---|---|
| | | Guselkumab | Histidine | Glycine | Metionine | Total aa | |
| 5-1 | 20 mM Ace, 4.7% Man (amino acid-free) | 0.0007 | | | | 0 | 1:0 |
| 5-2 | 20 mM Ace, 4.7% Man, 30 mM His | 0.0007 | 0.03 | | | 0.03 | 1:44 |
| 5-3 | 20 mM Ace, 4.7% Man, 30 mM Met | 0.0007 | | | 0.03 | 0.03 | 1:44 |
| 5-4 | 20 mM His, 4.7% Man | 0.0007 | 0.02 | | | 0.02 | 1:29 |
| 5-5 | 20 mM His, 4.7% Man, 30 mM His | 0.0007 | 0.05 | | | 0.05 | 1:73 |
| 5-6 | 20 mM His, 4.7% Man, 30 mM Met | 0.0007 | 0.02 | | 0.03 | 0.05 | 1:73 |
| 5-7 | 20 mM Pho, 4.7% Man(amino acid-free) | 0.0007 | | | | 0 | 1:0 |
| 5-8 | 20 mM Pho, 4.7% Man, 30 mM His | 0.0007 | 0.03 | | | 0.03 | 1:44 |
| 5-9 | 20 mM Pho, 4.7% Man, 30 mM Met | 0.0007 | | | 0.03 | 0.03 | 1:44 |

As a result, as shown in Table 24, similar to Table 17 above, it was confirmed that Formulations Nos. 5-2 to 5-6, 5-8, and 5-9, which are amino acid-containing formulations, contain 29 mol or more of amino acids per 1 mol of the antibody.

The foregoing description of the present disclosure is for illustrative purposes, and those skilled in the art to which the present disclosure pertains will understand that the present disclosure may be easily modified into other specific forms without changing the technical spirit or essential features thereof. Therefore, it should be understood that the embodiments described above are illustrative in all respects and not restrictive.

## Claims

1. A liquid formulation comprising:
an anti-IL-23 antibody;
a buffer; and
a stabilizer comprising at least one selected from sugars, sugar alcohols, amino acids, metal salts, salts thereof, and hydrates thereof;
wherein the liquid formulation has a pH of about 4.0 to about 8.5.

2. The liquid formulation of claim 1, wherein the anti-IL-23 antibody is an anti-IL-23p19 antibody.

3. The liquid formulation of claim 1, wherein the anti-IL-23 antibody is guselkumab.

4. The liquid formulation of claim 1, wherein the buffer comprises at least one selected from acetate, histidine, phosphate, citrate, succinate, malate, tartrate, carbonate, salts thereof, and hydrates thereof.

5. The liquid formulation of claim 1, wherein the buffer comprises at least one selected from acetate, histidine, phosphate, citrate, succinate, salts thereof, and hydrates thereof.

6. The liquid formulation of any one of claims 1 to 5, wherein the pH of the liquid formulation is about 4.0 to about 6.5.

7. The liquid formulation of claim 1, wherein the buffer comprises at least one selected from acetate, histidine, phosphate, salts thereof, and hydrates thereof.

8. The liquid formulation of claim 7, wherein the pH of the liquid formulation is about 4.0 to about 8.0.

9. The liquid formulation of claim 7, wherein the pH of the liquid formulation is about 5.5 to about 8.0.

10. The liquid formulation of claim 1, wherein the buffer comprises at least one selected from acetate, salts thereof, and hydrates thereof.

11. The liquid formulation of claim 10, wherein the pH of the liquid formulation is about 4.0 to about 6.5.

12. The liquid formulation of claim 10, wherein the pH of the liquid formulation is about 4.0 to about 5.2.

13. The liquid formulation of claim 1, wherein the buffer comprises at least one selected from histidine, a salt thereof, and a hydrate thereof.

14. The liquid formulation of claim 13, wherein the pH of the liquid formulation is about 4.8 to about 7.2.

15. The liquid formulation of claim 13, wherein the pH of the liquid formulation is about 5.6 to about 7.2.

16. The liquid formulation of claim 1, wherein the buffer comprises at least one selected from phosphate, salts thereof, and hydrates thereof.

17. The liquid formulation of claim 16, wherein the pH of the liquid formulation is about 5.8 to about 8.2.

18. The liquid formulation of claim 16, wherein the pH of the liquid formulation is about 5.8 to about 6.7.

19. The liquid formulation of claim 1, wherein the buffer comprises at least one selected from citrate, salts thereof, and hydrates thereof.

20. The liquid formulation of claim 19, wherein the pH of the liquid formulation is about 4.5 to about 7.0.

21. The liquid formulation of claim 19, wherein the pH of the liquid formulation is about 5.5 to about 6.5.

22. The liquid formulation of claim 1, wherein the buffer comprises at least one selected from succinate, salts thereof, and hydrates thereof.

23. The liquid formulation of claim 22, wherein the pH of the liquid formulation is about 4.3 to about 6.5.

24. The liquid formulation of any one of claims 1 to 23, wherein a concentration of the buffer is about 0.1 mM to about 150 mM.

25. The liquid formulation of any one of claims 1 to 23, wherein a concentration of the buffer is about 5 mM to about 50 mM.

26. The liquid formulation of any one of claims 1 to 23, wherein a concentration of the buffer is about 15 mM to about 100 mM.

27. The liquid formulation of claim 1, wherein the liquid formulation comprises at least one amino acid.

28. The liquid formulation of claim 1, wherein the liquid formulation comprises at least one amino acid in an amount of 20 moles or more per 1 mole of the antibody.

29. The liquid formulation of claim 1, wherein the sugars comprise at least one selected from trehalose, sucrose, galactose, mannose, maltose, lactose, fructose, and glucose.

30. The liquid formulation of claim 1, wherein the sugar alcohols comprise at least one selected from mannitol, sorbitol, xylitol, arabitol, erythritol, lactitol, maltitol, and inositol.

31. The liquid formulation of claim 1, wherein the amino acids comprise at least one selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

32. The liquid formulation of claim 1, wherein the metal salts comprise at least one selected from NaCl, KCl, NaF, KBr, NaBr, Na₂SO₄, NaSCN, CaCl₂, MgCl₂, and K₂SO₄.

33. The liquid formulation of claim 1, wherein a concentration of the sugars is about 0.1% (w/v) to about 20.0% (w/v).

34. The liquid formulation of claim 1, wherein a concentration of the sugar alcohols is about 0.1% (w/v) to about 20.0% (w/v).

35. The liquid formulation of claim 1, wherein a concentration of the amino acids is about 1 mM to about 400 mM.

36. The liquid formulation of claim 1, wherein a concentration of the metal salts is about 0.1% (w/v) to about 20.0%.

37. The liquid formulation of claim 1, wherein the stabilizer comprises at least one selected from sucrose, trehalose, mannitol, arginine, lysine, histidine, methionine, glycine, and NaCl.

38. The liquid formulation of claim 1, wherein the stabilizer comprises at least one selected from trehalose, mannitol, arginine, lysine, methionine, glycine, and NaCl.

39. The liquid formulation of claim 1, wherein the stabilizer comprises at least one selected from trehalose, mannitol, histidine, glycine, and methionine.

40. The liquid formulation of claim 1, wherein the stabilizer comprises at least one selected from trehalose, mannitol, glycine, and methionine.

41. The liquid formulation of claim 1, wherein the stabilizer comprises at least one selected from methionine, mannitol, and histidine.

42. The liquid formulation of claim 1, wherein the stabilizer comprises at least one selected from glycine, methionine, and mannitol.

43. The liquid formulation of claim 1, wherein the stabilizer comprises sucrose.

44. The liquid formulation of claim 43, wherein a concentration of the sucrose is about 0.1% (w/v) to about 20.0% (w/v).

45. The liquid formulation of claim 43, wherein a concentration of the sucrose is about 5.0% (w/v) to about 15.0% (w/v).

46. The liquid formulation of claim 43, wherein a concentration of the sucrose is about 8.0% (w/v) to about 12.0% (w/v).

47. The liquid formulation of claim 1, wherein the stabilizer comprises trehalose.

48. The liquid formulation of claim 47, wherein a concentration of the trehalose is about 0.1% (w/v) to about 20.0% (w/v).

49. The liquid formulation of claim 47, wherein a concentration of the trehalose is about 5.0% (w/v) to about 15.0% (w/v).

50. The liquid formulation of claim 1, wherein the stabilizer comprises mannitol.

51. The liquid formulation of claim 50, wherein a concentration of the mannitol is about 0.1% (w/v) to about 20.0% (w/v).

52. The liquid formulation of claim 50, wherein a concentration of the mannitol is about 2.0% (w/v) to about 8.0% (w/v).

53. The liquid formulation of claim 1, wherein the stabilizer comprises arginine.

54. The liquid formulation of claim 53, wherein a concentration of the arginine is about 1 mM to about 400 mM.

55. The liquid formulation of claim 53, wherein a concentration of the arginine is about 100 mM to about 200 mM.

56. The liquid formulation of claim 1, wherein the stabilizer comprises lysine.

57. The liquid formulation of claim 56, wherein a concentration of the lysine is about 1 mM to about 400 mM.

58. The liquid formulation of claim 56, wherein a concentration of the lysine is about 100 mM to about 200 mM.

59. The liquid formulation of claim 1, wherein the stabilizer comprises histidine.

60. The liquid formulation of claim 59, wherein a concentration of the histidine is about 1 mM to about 400 mM.

61. The liquid formulation of claim 59, wherein a concentration of the histidine is about 20 mM to about 100 mM.

62. The liquid formulation of claim 1, wherein the stabilizer comprises methionine.

63. The liquid formulation of claim 62, wherein a concentration of the methionine is about 1 mM to about 400 mM.

64. The liquid formulation of claim 62, wherein a concentration of methionine is about 20 mM to about 160 mM.

65. The liquid formulation of claim 1, wherein the stabilizer comprises glycine.

66. The liquid formulation of claim 65, wherein a concentration of the glycine is about 1 mM to about 400 mM.

67. The liquid formulation of claim 65, wherein a concentration of the glycine is about 200 mM to about 300 mM.

68. The liquid formulation of claim 1, wherein the stabilizer comprises NaCl.

69. The liquid formulation of claim 68, wherein a concentration of the NaCl is about 0.1% (w/v) to about 20.0%.

70. The liquid formulation of claim 68, wherein a concentration of the NaCl is about 0.1% (w/v) to about 5.0%.

71. The liquid formulation of any one of claims 1 to 70, further comprising a diluent.

72. The liquid formulation of claim 71, wherein the diluent is water.

73. The liquid formulation of any one of claims 1 to 72, wherein the liquid formulation inhibits aggregation of the antibody.

74. The liquid formulation of any one of claims 1 to 72, wherein the liquid formulation has an increased aggregation temperature (T_{agg}).

75. The liquid formulation of any one of claims 1 to 72, wherein the liquid formulation has an aggregation temperature (T_{agg}) of about 73.83 °C or higher.

76. The liquid formulation of claim 75, wherein the liquid formulation has an aggregation temperature (T_{agg}) of about 73.9 °C or higher.

77. The liquid formulation of claim 75, wherein the liquid formulation has an aggregation temperature (T_{agg}) of about 74 °C or higher.

78. The liquid formulation of claim 1, wherein the liquid formulation has an aggregation temperature (T_{agg}) of about 73.83 °C or higher, and is selected from the following:
A) the buffer comprises acetate, and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

79. The liquid formulation of claim 1, wherein the liquid formulation has an aggregation temperature (T_{agg}) of about 73.9 °C or higher, and is selected from the following:
A) the buffer comprises acetate, and the pH is about 4.0 to about 6.0; or
B) the buffer comprises histidine, and the pH is about 5.7 to about 7.0.

80. The liquid formulation of claim 1, wherein the liquid formulation has an aggregation temperature (T_{agg}) of about 73.83 °C or higher, and is selected from the following:
A) the stabilizer comprises trehalose;
B) the stabilizer comprises mannitol;
C) the stabilizer comprises arginine;
D) the stabilizer comprises lysine;
E) the stabilizer comprises histidine;
F) the stabilizer comprises methionine;
G) the stabilizer comprises glycine; or
H) the stabilizer comprises NaCl.

81. The liquid formulation of claim 1, wherein the liquid formulation has an aggregation temperature (T_{agg}) of about 74 °C or higher, and is selected from the following:
A) the stabilizer comprises trehalose;
B) the stabilizer comprises mannitol;
C) the stabilizer comprises methionine; or
D) the stabilizer comprises glycine.

82. The liquid formulation of any one of claims 1 to 72, wherein the liquid formulation has an increased diffusion interaction parameter (K_{D}).

83. The liquid formulation of any one of claims 1 to 72, wherein the liquid formulation has a diffusion interaction parameter (K_{D}) of about 20.0 mL/g or more.

84. The liquid formulation of claim 1, wherein the liquid formulation has a diffusion interaction parameter (K_{D}) of about 20.0 mL/g or more, and is selected from the following:
A) the buffer comprises acetate, and the pH is about 4.0 to about 6.0;
B) the buffer comprises histidine, and the pH is about 5.0 to about 7.0;
C) the buffer comprises phosphate, and the pH is about 6.0 to about 8.0;
D) the buffer comprises citrate, and the pH is about 4.5 to about 6.5; or
E) the buffer comprises succinate, and the pH is about 4.5 to about 6.0.

85. The liquid formulation of claim 1, wherein the liquid formulation has a diffusion interaction parameter (K_{D}) of about 21.0 mL/g or more, and is selected from the following:
A) the buffer comprises acetate, and the pH is about 4.0 to about 5.3; or
B) the buffer comprises histidine, and the pH is about 5.7 to about 7.0.

86. The liquid formulation of any one of claims 1 to 72, wherein the liquid formulation inhibits denaturation of the antibody.

87. The liquid formulation of any one of claims 1 to 86, wherein the liquid formulation is for subcutaneous injection.

88. A device comprising the liquid formulation of any one of claims 1 to 87.

89. The device of claim 88, comprising the liquid formulation in a container selected from a syringe, a pre-filled syringe, an auto-injector, a bottle, a vial, and a tube.

90. The device of claim 88, comprising the liquid formulation in a pre-filled syringe.

91. The device of claim 88, wherein the pre-filled syringe is a single-dose pre-filled syringe.

92. A method of treating an IL-23-related condition, comprising administering the liquid formulation of any one of claims 1 to 87 to a subject in need thereof.

93. The method of treating an IL-23-related condition of claim 92, wherein the IL-23-related condition is any one selected from psoriasis, psoriatic arthritis, palmoplantar pustulosis, Crohn's disease (CD), inflammatory bowel disease (IBD), multiple sclerosis (MS), ulcerative colitis (UC), and ankylosing spondylitis.

94. The method of treating an IL-23-related condition of claim 93, wherein the psoriasis is plaque psoriasis.

95. The method of treating an IL-23 related condition of claim 94, wherein the plaque psoriasis is moderate to severe plaque psoriasis.

96. The method of treating an IL-23 related condition of claim 93, wherein the psoriatic arthritis is active psoriatic arthritis (PsA).

97. The method of treating an IL-23 related condition of claim 93, wherein the ulcerative colitis is moderately to severely active ulcerative colitis.

98. A use of the liquid formulation of any one of claims 1 to 87 in the manufacture of a medicament for treating an IL-23-related condition.
